(19) Europäisches Patentamt
European Patent Office
Office européen des brevets

(11) **EP 3 390 663 B1**

(12) **EUROPEAN PATENT SPECIFICATION**

(45) Date of publication and mention
of the grant of the patent:
**17.04.2024 Bulletin 2024/16**

(21) Application number: **16823250.2**

(22) Date of filing: **19.12.2016**

(51) International Patent Classification (IPC):
**C12Q 1/68** $^{(2018.01)}$

(52) Cooperative Patent Classification (CPC):
**C12Q 1/6806; C12Q 1/6846;** C12Q 2527/101;
C12Q 2527/125; C12Q 2565/625

(86) International application number:
**PCT/EP2016/081748**

(87) International publication number:
**WO 2017/103269 (22.06.2017 Gazette 2017/25)**

(54) **METHOD FOR THE DETECTION OF A SEXUALLY TRANSMITTED INFECTIOUS PATHOGEN**

VERFAHREN FÜR DEN NACHWEIS VON SEXUELL ÜBERTRAGBAREN INFEKTIÖSEN
KRANKHEITSERREGERN

PROCÉDÉ DE DÉTECTION D'UN AGENT PATHOGÈNE INFECTIEUX SEXUELLEMENT
TRANSMISSIBLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.12.2015 DK 201500822**

(43) Date of publication of application:
**24.10.2018 Bulletin 2018/43**

(73) Proprietor: **Selfdiagnostics OÜ**
**10138 Tallinn (EE)**

(72) Inventors:
• **KRÖLOV, Katrin**
**51011 Tartu (EE)**
• **LANGEL, Ülo**
**113 59 Stockholm (SE)**
• **TULP, Indrek**
**50604 Tartu (EE)**

(74) Representative: **Aera A/S**
**Niels Hemmingsens Gade 10, 5th Floor**
**1153 Copenhagen K (DK)**

(56) References cited:
WO-A2-2015/063498    JP-A- 2000 279 195
KR-A- 20130 092 164    US-A1- 2015 322 493

• **ALISON J. MOORE ET AL: "Antimicrobial activity
of cecropins", JOURNAL OF ANTIMICROBIAL
CHEMOTHERAPY., vol. 37, no. 6, 1 January 1996
(1996-01-01), pages 1077-1089, XP055358384, GB
ISSN: 0305-7453, DOI: 10.1093/jac/37.6.1077**
• **JEKATERINA JEVTUSEVSKAJA ET AL:
"Combination with antimicrobial peptide lyses
improves loop-mediated isothermal
amplification based method for Chlamydia
trachomatis detection directly in urine sample",
BMC INFECTIOUS DISEASES, vol. 16, no. 1, 13
December 2016 (2016-12-13), XP055356927, DOI:
10.1186/s12879-016-1674-0**

Remarks:
The complete document including Reference
Table(s) and the Sequence Listing(s) can be
downloaded from the EPO website

**Description**

FIELD OF THE INVENTION

The invention is directed to compositions and method for rapid, robust and simple biological sample preparation that allows efficient nucleic acid amplification directly from crude biological sample without requirement for prior nucleic acid purification.

The present invention especially relates to methods for the detection of sexually transmitted infectious pathogens in human subjects, especially bacterial pathogens such as Chlamydia trachomatis, Neisseria gonorrhoeae and urine based E. coli. The detection can be done without sample purification. The methods are optimized for detecting of sexually transmitted bacteria in urine samples.

BACKGROUND OF THE INVENTION

Sexually transmitted diseases (STDs) are infections that you can get from having sex with someone who has the infection. The causes of STDs are bacteria, parasites and viruses. There are more than 20 types of STDs, including Chlamydia, Gonorrhea, Genital herpes, HIV/AIDS, HPV, Syphilis, and Trichomoniasis.

Chlamydia trachomatis is the most common cause of sexually transmitted diseases affecting both men and women. More than half of C. trachomatis-positive patients have minimal or no symptoms, providing an ongoing reservoir for the infection. The lack of rapid sensitive tests for C. trachomatis detection makes it difficult to diagnose Chlamydia infection efficiently.

Being an obligate intracellular pathogen makes it difficult to culture C. trachomatis in laboratory. Treatment of C. trachomatis infection depends not only on the site of the infection, but also on the patient's age and on the case of complexity. Thus, it is very important to identify C. trachomatis in the early stage of infection and start immediate treatment as soon as possible to prevent the development of further long-term complications and decrease the chance of getting other infections such as N. gonorrhoeae or Human immunodeficiency virus.

The traditional diagnosis of sexually transmitted Chlamydia infection involves collection of the samples: regularly urethral swab or urine specimen from males and endocervical or vaginal swab from females. Successful screening and preventing further spread and complications of Chlamydia depends on the ability to diagnose infections accurately, rapidly and inexpensively. Modern laboratories still use traditional testing methods such as cell culture and antigen based detection. The cell culture method is highly specific, but has very low sensitivity, is expensive, slow (the result could be obtained only after 3 days) and requires special sample collection, storage and transport. Immunological assays like the enzyme immunoassay and direct fluorescent antibody (DFA) assay have low sensitivity and specificity as a cell culture method, which limits the use of these tests in diagnostic field.

As an alternative to the traditional laboratory methods, nucleic acid amplification tests (NAATs) were developed allowing detection of pathogen-specific DNA or RNA sequences. These tests are significantly more sensitive for the screening and diagnosis of genital Chlamydia infection because they can detect as little as single nucleic acid copy of the target.

There are several different nucleic-acid amplification based tests for C. trachomatis detection: Abbott RealTime CT/NG uses ligase chain reaction (LCR); Aptima COMBO uses transcription-mediated amplification (TMA); BD ProbeTec ET use strand displacement amplification (SDA); Xpert CT/NG and Cobas CT/NG tests use real-time polymerase chain reaction (PCR).

There are several drawbacks to these types of methods, including that they cannot be used by individuals without expertise within the field or without the use of for example a PCR machine or sample preparation.

It is therefore clear that there is a need for tests against sexually transmitted diseases that requires little or no sample preparation, a simple test setup, and little or no expertise within the technical field. Such test could for example be an over the counter do-it-yourself kit.

WO 98/11259 describes the testing of genitourinary body fluid samples using a nucleic acid amplification technique (NAAT) to detect a plurality of sexually transmitted disease pathogens including Chlamydia trachomatis, Neisseria gonorrhoeae, Mycoplasma genitalium and Ureaplasma urealyticum.

WO 2011/095888 relates to a method and device for the detection of target molecules from a sample in rapid tests.

WO 2014/060604 relates to a method for rapid sample pre-treatment and amplification directly from crude biological sample lysates.

WO 2011/038197 relates to a method for detecting a target nucleic acid species by isothermal amplification on e.g. Chlamydia by subjecting the cells to a lytic enzyme. In the present context, the enzyme breaks the bacterial cell wall via a catalytic reaction. WO 2011/038197, do not provide the skilled person with any alternative means for the lysis.

## SUMMARY OF THE INVENTION

An antimicrobial peptide like cecropin as disclosed by the present inventors does not catalyse a chemical reaction, but acts via a transmembrane potential and penetrates the cell wall, thereby disturbing the wall and creating pores/holes into it. Such an alternative approach for detecting a target nucleic acid species provides significant benefits for adapting diagnostics to point of care devises, which do not have the means for sample purification, and wherein the amplification must be applied on the crude sample material.

The present invention relates to a method for the detection of a sexually transmitted infectious pathogen such as bacteria in a human subject without sample purification,

the method comprising the steps;

providing a urine sample from the human subject,
adding a cecropin to the urine sample, amplifying the released nucleic acids by loop-mediated isothermal amplification (LAMP) using primers targeting the sexually transmitted infection pathogen nucleic acids,
detecting a signal from the nucleic acid originating from the sexually transmitted infection pathogen organism,

and indicating the human subject being infected with a sexually transmitted infectious pathogen, if the signal is above a predetermined value.

In one embodiment of the present invention, the urine sample is diluted more than 50%.

In another embodiment of the present invention, the total assay time is less than 60 minutes.

In a further embodiment of the present invention, the antimicrobial peptide based release of nucleic acids is combined with a heat treatment.

In yet another embodiment of the present invention, the LAMP reactions are analysed or displayed in a lateral flow strip.

In a further embodiment of the present invention, the sexually transmitted infection pathogen primers are designed to detect Chlamydia trachomatis specific nucleic acids.

In yet another embodiment of the present invention, the antimicrobial peptide is a cecropin.

In a further embodiment of the present invention, the sensitivity is higher than 70%.

## DETAILED DESCRIPTION OF THE INVENTION

In its broadest aspect, the present invention relates to point of care methods for detecting sexually transmitted infectious pathogens. Shifting the molecular biological platform for diagnosis from highly complex laboratories' workflow to simple point of care devices, such as microfluidic devices is a perilous process. Thus, in one embodiment the present invention enables early stage indication, because the subject can actually indicate himself or herself as infected or not.

In one embodiment of the present invention, the indication is a reliable diagnosis.

The present invention provides means for improved test accuracy, ease of specimen management, convenience in specimen management, and ease of screening sexually active men and women.

The present invention provides means for simple indication or diagnosis that can be done outside a laboratory.

The present invention is an easy do-it-yourself kit for an indicator of whether an individual should seek medical attention.

In the example section, the present inventors present a rapid and sensitive assay based on loop-mediated isothermal amplification method (LAMP), which can detect at least 5 C. trachomatis pathogens per reaction (25 cryptic plasmid copies) from urine samples within 21 min, and the reaction product can be detected using lateral-flow (LF) strips, which is very beneficial for application in the point-of-care (POC) settings.

The clinical data showed that specificity of the assay is 100 % and sensitivity 73 %. Such high percentages' level is necessary, if your objective is to provide a reliable diagnostic point of care method, thus to the skilled addressee this is an issue, which requires significant testing, because a POC method cannot rely on e.g. trained staff or sophisticated equipment.

Additionally, it is demonstrated that the present assay does not give any cross-reactivity with the more than 30 pathogen's DNA potentially present in the urine samples.

Furthermore, the assay does not require purification or extraction of DNA from clinical sample prior to amplification, so the need for specialized equipment is eliminated.

The assay allows detection of C. trachomatis directly from urine sample, providing rapid, sensitive, diagnosis with minimal need for instructions. The very same assay conditions, except primers, allows detection of Neisseria gonorrhoeae directly from urine sample, providing rapid, sensitive, diagnosis with minimal need for instructions.

This makes the whole procedure significantly less laborious, less time-consuming and consequently less expensive for early detection and identification of infectious disease.

All the above is the perilous yet necessary development required to implement such diagnostic assays in cost effective Point of Care method.

The present C. trachomatis and N. gonorrhoeae specific LAMP assay is optimised for its user interface, so it's simple to perform, and could therefore be applied in numerous point-of-care settings.

Thus, in one aspect, the present invention relates to a method of treating a urine sample with a pathogenic specific antimicrobial drug in order to release nucleic acids from the pathogen in the urine sample, and afterwards detect the presence of the pathogen.

One aspect of the present invention relates to a method for the detection of a sexually transmitted infectious pathogen in a human subject without sample purification, the method comprising the steps;

providing a urine sample from the human subject,

adding a cecropin to the urine sample,

amplifying the released nucleic acids by loop-mediated isothermal amplification (LAMP) using primers targeting the sexually transmitted infection pathogen nucleic acids,

detecting a signal from the nucleic acid originating from the sexually transmitted infection pathogen organism,

and indicating the human subject being infected with a sexually transmitted infectious pathogen, if the signal is above a predetermined value. The method is easily applicable for point-of-care devices while meeting such device requirements like speed (less than 60 minutes), efficiency (sensitivity of >60% and specificity >95%) and usability (do not require sophisticated equipment or medical training).

In one embodiment, the present invention relates to a method for the detection of a sexually transmitted infectious pathogen in a human subject comprising

a) providing a urine sample from the human subject,
b) adding a cecropin to the urine sample to release nucleic acids from the cells in the sample with the proviso that no further nucleic acid purification applied,
c) amplifying the released nucleic acids by loop-mediated isothermal amplification (LAMP) using primers targeting the sexually transmitted infection pathogen nucleic acids, and
d) detecting the amount of nucleic acid originating from the sexually transmitted infection pathogen organism

thereby indicating the likelihood of the human subject being infected with a sexually transmitted infectious pathogen.

In another embodiment, the present invention relates to a method for determining the presence of a sexually transmitted infectious pathogen in a human subject comprising

a) contacting a urine sample obtained from the human subject a cecropin to release nucleic acids from the pathogen cells in the urine sample
b) amplifying the un-purified released nucleic acids from the pathogenic cells by a loop-mediated isothermal amplification (LAMP) method comprising primers from nucleic acids of a known sexually transmitted infectious pathogen,
c) determining presence in the sexually transmitted infectious pathogen in the urine sample, by detecting amplification of the released nucleic acids.

In another embodiment, the present invention relates to a method for determining the presence of a sexually transmitted infectious pathogen in a human subject comprising

a) contacting a urine sample obtained from the human subject with a cecropin
b) amplifying the un-purified released nucleic acids from the pathogenic cells by loop-mediated isothermal amplification (LAMP)comprising primers targeting nucleic acids of asexually transmitted infectious pathogen,
c) determining presence in the sexually transmitted infectious pathogen in the urine, by detecting amplification of the nucleic acids from the sexually transmitted infectious pathogen.

One presently preferred aspect of the invention relates to a point-of-care method for the detection of Chlamydia trachomatis in a human subject without sample purification, the method comprising the steps;

a) providing a urine sample diluted more than 50% from the human subject,

b) adding a Cecropin peptide to the urine sample,

c) amplifying the released nucleic acids by loop-mediated isothermal amplification (LAMP) using primers targeting Chlamydia trachomatis nucleic acids using a Bsm polymerase,

d) detecting a signal from the nucleic acid originating from Chlamydia trachomatis by a lateral flow strip, and

e) indicating the human subject being infected with Chlamydia trachomatis, if the signal is above a predetermined value,

wherein said point-of-care method have a detection sensitivity of more than 60% compared to the Cobas®4800 CT/NG Test (Roche) in standard settings.

Another presently preferred aspect of the invention relates to a point-of-care method for the detection of Neisseria gonorrhoeae in a human subject without sample purification, the method comprising the steps;

a) providing a urine sample diluted more than 50% from the human subject,

b) adding a Cecropin peptide to the urine sample,

c) amplifying the released nucleic acids by loop-mediated isothermal amplification (LAMP) using primers targeting Chlamydia trachomatis nucleic acids using a Bsm polymerase,

d) detecting a signal from the nucleic acid originating from Chlamydia trachomatis by a lateral flow strip, and

e) indicating the human subject being infected with Chlamydia trachomatis, if the signal is above a predetermined value,

wherein said point-of-care method have a detection sensitivity of more than 60% compared to PCR based standard settings.

Another presently preferred aspect of the invention relates to a point-of-care method for the detection of Chlamydia trachomatis and/or Neisseria gonorrhoeae in a human subject without sample purification, the method comprising the steps;

a) providing a urine sample,

b) adding a Cecropin peptide to the urine sample, and allow the peptide to lysis the cells for less than 15 minutes,

c) diluting the urine sample obtained in step b) more than 50%

d) amplifying the released nucleic acids by loop-mediated isothermal amplification (LAMP) using primers targeting Chlamydia trachomatis and/or Neisseria gonorrhoeae nucleic acids using a Bsm polymerase,

e) detecting a signal from the nucleic acid originating from Chlamydia trachomatis and/or Neisseria gonorrhoeae by a lateral flow strip, and

f) indicating the human subject being infected with Chlamydia trachomatis and/or Neisseria gonorrhoeae, if the signal is above a predetermined value,

wherein said point-of-care method have a detection sensitivity of more than 60% compared to the Cobas®4800 CT/NG Test (Roche) in standard settings.

*Sexually transmitted infectious bacteria*

Sexually transmitted infections (STI), also referred to as sexually transmitted diseases (STD) and venereal diseases (VD), are infections that are commonly spread by sex.

Most STIs initially do not cause symptoms. This results in a greater risk of passing the disease on to others. The present invention reduces the risk of passing on the STIs to others by providing a simple, fast and reliable test for detecting sexually transmitted pathogens.

In the present context, the terms STI, STD, VD, pathogens and sexually transmitted pathogens (STP) are used interchangeably, and they also cover urinary tract infections (UTIs).

More than 30 different bacteria, viruses, and parasites can cause STIs. Bacterial STIs include chlamydia, gonorrhea, and syphilis among others. There are many species of bacteria, protozoa, fungi, and viruses, many that remain undocumented or poorly studied. Despite that, sexually transmission of microbes is far from limited to the above. Since the sexual route of transmission is not considered common, and/or the microbe itself is not implicated in a major research study on disease, the following pathogens are simply not screened for in sexual health clinics. Some of these microbes are known to be sexually transmittable.

Microbes known to be sexually transmissible (but not generally considered STDs/STIs) include: Marburg virus and HTLV (both types 1 and 2).

In one embodiment, the present invention relates to any pathogen being present at any time in urine and/or at any time occupies the urethra.

In a presently preferred embodiment, the pathogen is bacteria.

In another embodiment, the pathogen is a gram-negative bacterium.

In one embodiment, the bacteria selected group consisting of Haemophilus ducreyi, Chlamydia trachomatis, Neisseria gonorrhoeae, Klebsiella granulomatis, Mycoplasma genitalium, Mycoplasma hominis, Treponema pallidum, Ureaplasma urealyticum and Ureaplasma parvum.

In another embodiment, the bacteria selected group consisting of Chlamydia trachomatis, Neisseria gonorrhoeae, urine based E. Coli and Mycoplasma genitalium.

*Chlamydia*

In one embodiment of the present invention, the sexually transmitted pathogen is Chlamydia trachomatis.

Chlamydia infection is a common sexually transmitted infection in humans caused by the bacterium Chlamydia trachomatis. The term Chlamydia infection can also refer to infection caused by any species belonging to the bacterial family Chlamydiaceae.

C. trachomatis is found only in humans. Chlamydia is a major infectious cause of human genital and eye disease.

In most cases, Chlamydia infection is asymptomatic and therefore can remain untreated for prolonged periods.

C. trachomatis is a gram-negative, obligate intracellular human pathogen. C. trachomatis strains are serologically classified into 15 serovars based on antigenic variation of the major outer membrane protein. A-C serovars are eye pathogens causing ocular trachoma. Serovars D-K and lymophogranuloma venereum serovars L1-L2 are sexually transmitted pathogens that infect columnar epithelial cells of the genital tract.

In one embodiment of the present invention, the sexually transmitted pathogen is Chlamydia trachomatis having Serovars D-K and lymophogranuloma venereum serovars L1-L2.

The present invention can detect the serological strain, which causes the infection and thereby enables correct indication, diagnosis and subsequent treatment.

In another embodiment of the present invention, the sexually transmitted pathogen is a bacterium belonging to bacterial family Chlamydiaceae.

Chlamydia is known as the "silent epidemic" in women, it may not cause any symptoms in 70-80% of cases, and can linger for months or years before being discovered. The present invention provides means for detection in individuals that may not know that they are infected with chlamydia bacteria, and therefore do not seek medical attention.

*Gonorrhea*

Gonorrhea, also known as gonnococcal infection, gonococcal urethritis, gonorrhoea and the clap, is a sexually transmitted infection that is caused by the bacterium Neisseria gonorrhoeae.

Neisseria gonorrhoeae is a Gram-negative coffee bean-shaped diplococci bacteria.

In one embodiment of the present invention, the sexually transmitted pathogen is Neisseria gonorrhoeae.

Neisseria gonorrhoeae PCR-based testing require a relative high level of training and complex thermo-cycling machines, therefore not something that can easily be done outside a laboratory.

However, as described above the present invention provides means for detection Neisseria gonorrhoeae in individuals that may not know that they are infected with gonorrhea, and therefore do not seek medical attention.

However, a selection of appropriate primer set for N. gonorrhoeae is not trivial task. Good target region for detection often may give cross-reactivity with other Neisseria spices that are not under interest. This data is provided on Example 7, where for example primer sets developed for 16S rRNA region gave cross-reactivity (false positive results in perspective of diagnosis) with other spices like *N. cinerea, N. flava, N. meningitidis* (serogroup B), *N. meningitidis* (serogroup Y), *N. lactamica* and *N. perflava.* While primer sets developed for 2086 region have cross-reactivity with only *N. lactamica* that is rarely found in urine, presumably only in certain timeframe after oral sex.

*Urine based E. Coli*

In one embodiment of the present invention, the pathogen is a urine based E. Coli.

E. coli is a Gram-negative, rod-shaped bacterium that is commonly found in the lower intestine of warm-blooded organisms (endotherms). Some E. coli such as but not limitned to enteropathogenic E. coli (EPEC), Shiga toxin-producing E. coli (STEC, Shigella/enteroinvasive E. coli (EIEC), enteroaggregative E. coli (EAEC), diffusely adherent E. coli (DAEC), enterotoxigenic E. coli (ETEC), and adherent invasive E. coli (AIEC) are pathogenic.

It is an object of preferred embodiments of the present invention to be able to detect the E. coli species that causes infection in a urine sample. The present invention can detect the species-specific origin of urinary tract infection (UTI) caused by any of the above-mentioned E. coli.

In the elderly and the very young, UTI symptoms may be vague or non-specific. The main causal agent of both types is Escherichia coli (E. coli), but UTIs can also be cause by other bacteria such as chlamydia or gonorrhea.

In one embodiment, the method of the present invention can differentiate a urinary tract infection cause by Escherichia coli, Chlamydia trachomatis and/or Neisseria gonorrhoeae. The rapid distinguishing between the 3 main causes of urinary tract infection is an advantage of the present invention.

*Urine sample*

A "sample," as used herein, includes urine samples (including samples derived from urine samples), endocervical swabs, vaginal swabs, urethral swabs, and rectal swabs.

The term sample also includes diluted and/or buffered forms of the above samples, for example, a buffer into which a swab sample has been placed, a urine sample to which a buffer has been added, and the like.

In a presently preferred embodiment, the sample is a urine sample.

Sample collection can be performed by subjects themselves without intervention by the healthcare professional. The urine sample collected for the methods of present invention can be collected at different points in time and be or different sizes.

Preferably, the sample will be of first pass urine, also known as first catch urine, since this portion of urine voided by a the human subject, which is not diluted by midstream urine may contain the greatest concentration of pathogens from the urethra.

The best sample is usually first-void morning urine, because it contains more pathogens or bacteria.

The sample may be fresh or frozen.

For fresh urines native, first-catch urine should preferably be collected and immediately used.

Alternatively, they can be stored, but preferably not longer than 2h at RT or 24h at +4°C.

Preferably, the stored urine sample is treated beforehand with 10 mM EDTA as soon as possible to avoid DNA degradation by nucleases.

Optionally samples can be stored below -20°C for later use as frozen samples.

In some embodiments, the same results can be achieved with a urethral swab sample, a vaginal swab sample, or an endocervical swab sample.

*Dilution of urine sample*

The examples herein show that the dilution of the urine sample may be important to enable the specific polymerase in the amplification step to amplify the target.

In the data provided by the present inventions, e.g. the Bsm polymerase in LAMP can tolerate easily up to 20% of urine in the reaction mixture without significant effect on reaction speed and thus also on sensitivity.

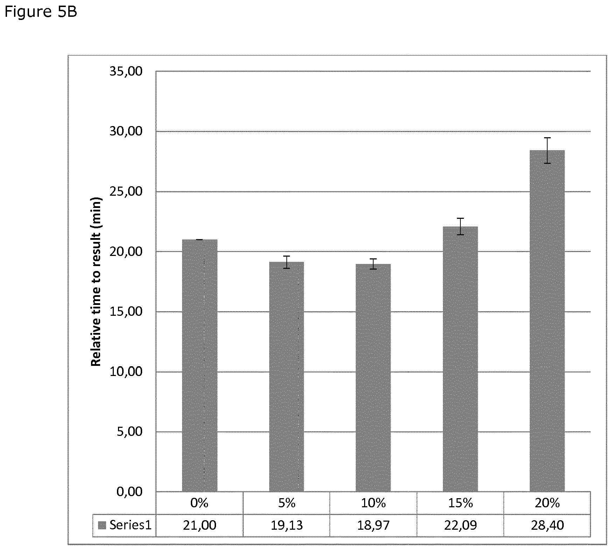

As it is shown on figure 5B, smaller amounts (5-10%) of urine even enhances the polymerase activity in comparison of pure water. This suggests that 10% of urine is absolute optimum in respect of reaction speed. Urine amount more than 15% starts to significantly to reduce polymerization reaction exponentially. Extrapolating provided data with power function

$$T_R = 0.0556u_\%^2 - 0.761u_\% + 21.32$$

where $T_R$ is relative time to result and $u_\%$ is urine precentral amount, results that more than 50% of urine concentration will lead to more than 2 hours reaction time, which is not feasible anymore for point-of-care applications. Generally, it is expected to have less than 1 hour turnaround time for point-of-care applications/devices.

With higher urine concentrations, the amplification reaction time should be extended otherwise loss in sensitivity may occur due to fact that exponential phase of the reaction is not yet reached or passed, and there will not be enough reaction products (amplicons) to detect.

For example, if the predetermined reaction time is 25 minutes and urine amount is 20%, then the reaction will not reach a sufficient level. This will result in no amplicons meaning that it will be misinterpreted as negative sample and through that the results will affect overall sensitivity. Thus, in a presently preferred embodiment, the amplification is provided by a polymerase capable of tolerating diluted urine, and preferably diluted more than 50%.

Thus, the present invention relates to a urine sample which has been diluted more than 50% with a buffer, such as but not limited to diluted more than 55%, diluted more than 60%, diluted more than 65%, diluted more than 70%, diluted more than 75%, diluted more than 80%, diluted more than 85%, diluted more than 90%, or diluted more than 95% prior to amplification of the target sequences.

*Amplification step*

The Loop-mediated isothermal amplification (LAMP) method as described herein stands out to be a novel, highly sensitive and specific diagnostic tool because of the ease of performing and capability to diagnose a negligible amount of pathogen genetic material within an hour.

The LAMP method of the present invention has several advantages over the widely-used PCR.

The LAMP method of the present invention is carried out at a constant temperature (typically 55-65°C) and does not require a thermal cycler. As shown in the Examples the temperature is typically around 60°C, such as 55-65°C, or 58-63°C. In some embodiments, the temperature is 55°C, 56°C, 57°C, 58°C, 59°C, 60°C, 61°C, 62°C, 63°C, 64°C and/or 65°C

The LAMP method of the present invention uses 4-6 different primers (2 inner, 2 outer and/or 2 additional loop primers) and a polymerase with a strand displacement activity to identify 6 distinct regions on the target gene sequence. This allows generating an amplification product containing of single-stranded loop regions, where primers can bind without template denaturation. The additions of loop primers significantly accelerate amplification, increasing sensitivity and reducing reaction time.

The amount of DNA product at the end of the LAMP reaction of the present invention is considerably higher that than in PCR and can be simply visualized using metal ion indicators like calcein or such DNA biding dyes as SYBR green, ethidium bromide, picogreen, propidium iodide, hydroxy naphthol blue.

Depending on primer choice, not only DNA but also RNA can be amplified. Thus, even pathogen specific RNA can be targeted as well with current invention.

*Polymerases*

The LAMP method of the present invention is using elevated temperature (usually between 55-65°C) and for that, a thermophilic DNA polymerase is required. These polymerases should have a strong strand displacement activity. These polymerases are not suitable for use in PCR. Not all thermostable DNA polymerase are suitable for urinary analysis.

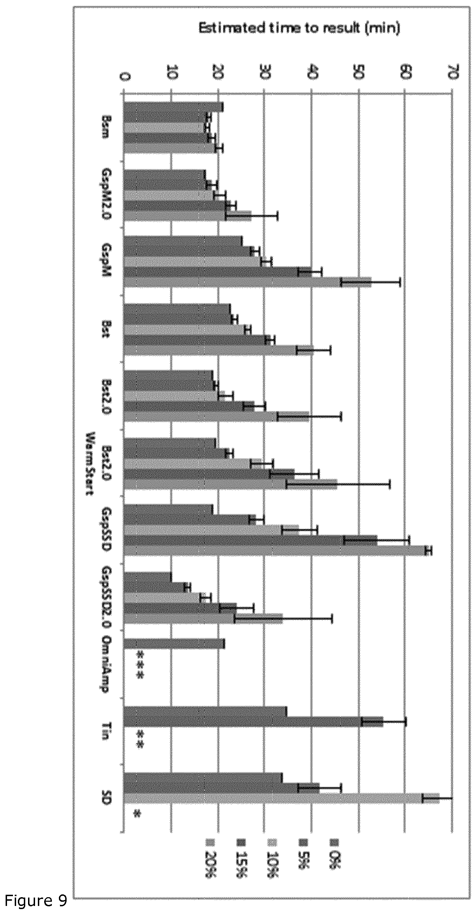

For urinary analysis, it is important to use a polymerase, which have minimum activity loss due urine matrix effect. On Figure 9 is provided a comparison of urine tolerance of different polymerases. Particularly Omi Amp, Tin and SD polymerases lose their activity entirely already on low levels of urine. Other polymerases under current study start to lose their activity significantly on urine levels 10% and more. While Bsm polymerase is most resistant to urine.

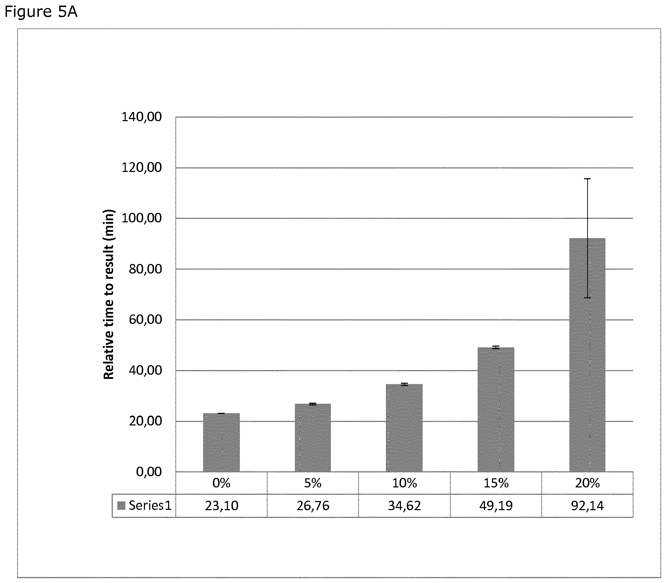

The present inventors reviewed numerous polymerases, and on Figure 5A is shown the urine tolerance of Bst polymerase and on Figure 5B is shown the tolerance for Bsm polymerase.

Small quantities of urine even enhance Bsm polymerase activity and thus this polymerase is very suitable for urinary analysis.

In a presently preferred embodiment of the present invention, the polymerase used in the LAMP is a Bsm polymerase. In the present context, a Bsm polymerase is a DNA polymerase of *Bacillus smithii,* which catalyzes 5'→3' synthesis of DNA and lacks 5'→3' and 3'→5' exonuclease activities.

In another embodiment, then the Bsm polymerase is a Bsm DNA Polymerase, Large Fragment. In the present context, a Bsm DNA Polymerase, Large Fragment is a portion of DNA polymerase of Bacillus smithii, which catalyzes 5'→3' synthesis of DNA and lacks 5'→3' and 3'→5' exonuclease activities. Bsm DNA Polymerase, Large Fragment has a strong strand displacement activity and is active in a wide range of temperatures from 30°C to 63°C, with an optimum of activity at 60°C. Bsm DNA Polymerase, Large Fragment is an enzyme with high functional similarity to Bst DNA Polymerase, Large Fragment and can replace it in most applications.

*Antimicrobial peptides*

Antimicrobial peptides (AMPs) are a unique and diverse group of molecules, which are divided into subgroups on the basis of their amino acid composition and structure. Currently, over 2500 AMPs have been described, of synthetic or natural origin, isolated from a variety of organisms in the Antimicrobial Peptide Database, on the University of Nebraska Medical Centers homepage.

The key feature for the antimicrobial peptides of the present invention is their ability to release nucleic acids from pathogens and suppress inhibitory effect caused by sample matrix. Usually such peptide will disrupt the cell membrane and thus causing lysis. This release allows better and easier detection of the presence of the bacteria or pathogen. Efficient lysis concentration for most peptides will be in the range of 5-100 $\mu$M concentration; however, the efficient lysis concentration can be smaller or larger depending on the peptide and pathogen target.

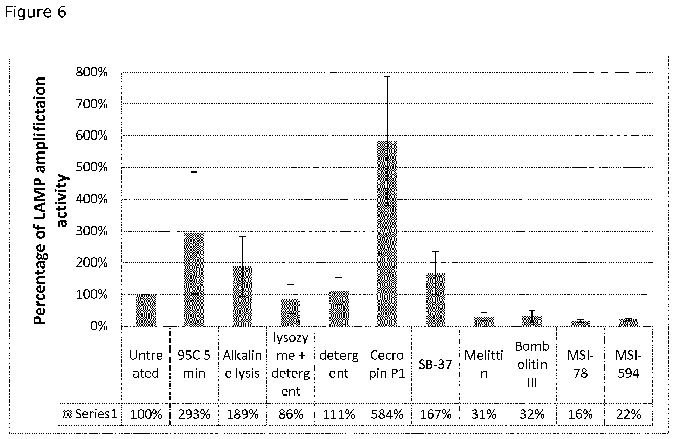

As shown in the examples of the present invention, the pre-treatment of the urine samples with antimicrobial peptides gave up to 6 times increased amount of target DNA compared to thermal and alkaline sample preparation (Figure 6). Thus, the new sample pre-treatment method of the present invention is well suitable with downstream amplification applications necessary for adapting the methods to POC.

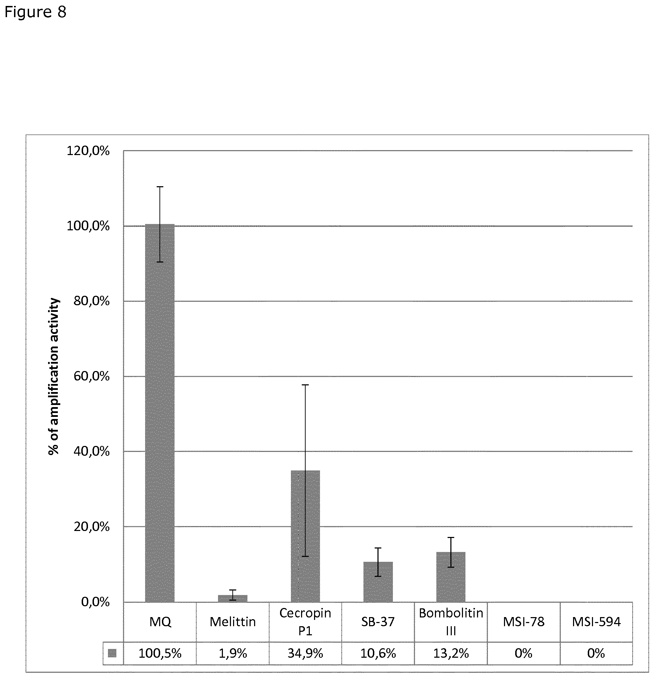

The use of peptides with higher concentrations will inhibit the amplification reaction in certain extent in comparison of ideal conditions, e.g. pure water (MQ) (Fig. 8.). This is a drawback of use of peptides. However, not all the peptides have an equal effect. Among current selection of peptides, Cecropin P1 has a lowest inhibitory effect and therefore it has highest potentiality for the application(s) described herein. The right concentration of use needs to be balanced taking into account a sample matrix effect. As it was shown on Fig. 6., in urinary sample types specifically Cecropin P1 have even enhancing effect in comparison of pure urinary sample. This effect is explained by secondary property of peptides, which is an anti-inhibitory effect. Meaning, that in additionally to lysis effect, peptides also neutralizing inhibitory substances in real samples.

The present inventors suggest not to use too high peptide concentrations, as some peptides could have an inhibiting effect on the downstream application e.g. the LAMP reaction, thus in a presently preferred embodiment, the AMP concentration is in the range of 1-100 $\mu$M antimicrobial peptides in an undiluted urine sample, such as but not limited to 1-90 $\mu$M antimicrobial peptides, 10-80 $\mu$M antimicrobial peptides, 15-50 $\mu$M antimicrobial peptides, 20-40 $\mu$M antimicrobial peptides, and/or 30-40 $\mu$M antimicrobial peptides.

Most of AMPs have membrane active properties possibly forming pore like structures to make membrane permeable (mastoporan, melittin, bombolitin, magainins) or acting through the carpet model to disrupt microorganism membranes (cecropins).

Magainins are thought to permeabilize cell membranes by forming toroidal-pores. The proposed mechanism of action of cecropins involve the initial binding of the peptide through electrostatic attraction. It is suggested that these AMPs interact with the lipid portion of the cell membrane forming ion-permeable pores.

In one embodiment, the present invention relates to AMPs having linear cationic a-helical peptides.

In another embodiment, the present invention relates to AMPs lacking cysteine.

The antimicrobial peptides used in the methods of the present invention can be natural or synthetically antimicrobial peptides.

In one embodiment, the antimicrobial peptides of the present invention is selected from the group consisting of cecropins, mastoporan, magainins, melittines, and bombolitines.

In one embodiment, the antimicrobial peptides is selected from the group consisting of mastoporan, magainins, melittines, and bombolitines.

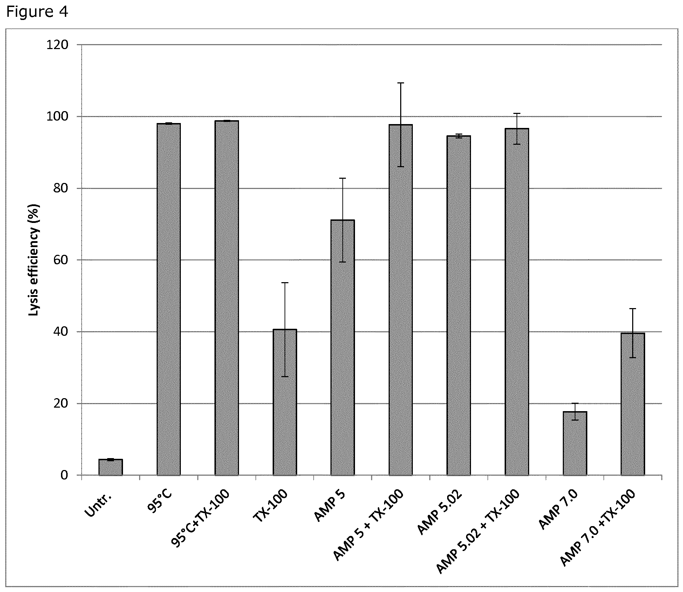

In a presently preferred embodiment, the antimicrobial pepetides are cecropins. Although there are also other peptides that that show relevant lysis effectiveness (Figure 4, AMP 5.02 represents Cecropin P1), they also significant inhibitory effect to LAMP reaction as represented on Figure 6.

*Cecropins*

Cecropins are small proteins of typically about 31 - 37 amino acid residues active against both Gram-positive and Gram-negative bacteria.

Cecropins isolated from insects other than Hyalophora cecropia (Cecropia moth) have been given various names; bactericidin, lepidopteran, sarcotoxin, etc. All of these peptides are structurally related.

Cecropin P1, an intestinal antibacterial peptide from Sus scrofa (Pig), also belongs to this family.

Cecropin family also consists Cecropin A and Cecropin B.

Cecropins possess antimicrobial activity against a wide variety of bacteria through compromising membrane permeability.

In one embodiment of the present invention, the Cecropins may be modified or being a derivate.

In one presently preferred embodiment, the AMP is a Cecropin.

The present inventors tested antimicrobial peptides cecropins (P1 and SB-37) which both resulted in significantly increased DNA levels. SB-37 gained on average 2 times increase of the target DNA. The peptide sequence of Cecropin B analogue SB-37 is MPKWKVFKKIEKVGRNIRNGIVKAGPAIAVLGEAKALG.

Cecropin P1 was most efficient of all tested treatments, gaining on average 6 times increase of the DNA available for the isothermal amplification. Thus, in a specially preferred embodiment, the Cecropin is Cecropin P1. The peptide sequence of Cecropin P1 is SWLSKTAKKLENSAKKRISEGIAIAIQGGPR.

Sample pre-treatment with other antimicrobial peptides did not give significant increase in isothermal amplification efficiency, but still show efficacy. The lack of increase in the isothermal amplification efficiency is probably due to inefficient lysis in case of melittin and bombolitin III, or inhibition of the amplification in case of Magainin group peptides (MSI-78 and MSI-594).

SEQ ID NO # 1

MPKWKVFKKIEKVGRNIRNGIVKAGPAIAVLGEAKALG
Hyalophora cecropia
Cecropin SB37

SEQ ID NO # 2

SWLSKTAKKLENSAKKRISEGIAIAIQGGPR
Ascaris suum,
Cecropin P1

*Melittin*

Melittin is the main peptide component of the honeybee venom. It shows potent hemolytic and cytolytic activities not only against mammalian cells but owns a broad spectrum of antibacterial, antifungal, antiviral, and antiprotozoal properties.

Melittin incorporates into cell membranes forming pore-like structures that lead to profound compromise of the cell permeability and following cell death.

In one embodiment, the AMP is a Melittin.

*Bombolitin*

Bombolitin is the most abundant component of bumblebee venom that share structure and biological properties with melittin. Bombolitins have broad range of activity against Gram-negative and Gram-positive bacteria, as well as plant pathogenic fungi, erythrocytes, mast cells and liposomes.

In one embodiment, the AMP is a Bombolitin.

*Magainins*

Magainins were initially isolated from the skin of the frog Xenopus laevis. Magainin analogues MSI-78 and MSI 594 are a very potent AMP being active against numerous bacterial strains, including those strains that are resistant to conventional antibiotics.

In one embodiment, the AMP is a Magainin.

*Lysis mixture (cocktail)*

Using developed lysis mixture (cocktail) that consists in urine (treated) in ranges:

0.5 - 5$\mu$M AMP (such as Cecropin P1)
5 - 50mM EDTA
0.1 - 10% non-ionic surfactant

The Lysis cocktail consists of an antimicrobial peptide (preferably Cecropin P1) that specifically lyses gram negative bacteria (in particular Chlamydia trachomatis and Neisseria gonorrhoeae), EDTA that is important to inhibit endonucleases, and a non-ionic surfactant that is important to lyse mammalian host cells, specifically important for lysis of Chlamydia trachomatis because it is an intracellular parasite. In the Examples, the inventors have used a Triton X-100, but other non-ionic surfactant that has a hydrophilic polyethylene oxide chain and an aromatic hydrocarbon lipophilic or hydrophobic group works.

Abovementioned concentrations are calculated for the diluted urine samples of the present invention.

Concentration of components can be varied and balanced per sample type and dilution ratio. For instance, samples that have higher cellular material contact may need higher ratio of lysis and inhibition supressing components and thus may also need a higher dilution ratio.

*Nucleic acid purification*

Nucleic acid purification is not necessary for the methods or the present invention. The fact that the methods presented herein does not require purification or extraction of DNA from clinical sample prior to amplification, provide that the need for specialized equipment is eliminated. This makes the whole procedure significantly less laborious, less time-consuming and consequently less expensive for early detection and identification.

Thus, in one embodiment of the present invention, no nucleic acid purification is performed.

However, filtration or up-concentration can be done by simple means, and can in specific embodiments be relevant to increase sensitivity. In such an embodiment, the sensitivity is increased via pre-concentrating targeted DNA amount and thus those samples that have DNA load below assay LOD (limit of detection) will be detected instead of considered false negative.

*Primers*

The primers used in the methods of the present invention are designed to be species specific to the bacteria to be tested. The primers can e.g. be specific to a species of bacteria or a genus, depending on what outcome is desired.

In one embodiment, the primers are designed to detect Chlamydia genus specific nucleic acids.

In another embodiment, the primers are designed to detect Chlamydia trachomatis species-specific nucleic acids.

In a presently preferred embodiment, the Chlamydia trachomatis primers are the LAMP primers listed in Table S1.

In one embodiment, the primers are designed to detect Neisseria gonorrhoeae specific nucleic acids given in Tables 1-3.

In a further embodiment, primers are designed to detect E. coli specific nucleic acids.

It is also possible to simultaneously detect several types of bacteria in the same sample using multiple pathogen primer sets. In this case, the assay is a multiplex assay.

Some primer sets can give non-specific signal on lateral flow strips without prior amplification (possibly due to formation of heterodimers). These primer sets should be avoided, when lateral flow strip detection is performed after amplification.

The presently preferred primers of the present invention are

SEQ ID NO # 5

AATATCATCTTTGCGGTTGC
Chlamydia trachomatis
CT LAMP primer F3

SEQ ID NO # 6

TCTACAAGAGTACATCG GTCA
Chlamydia trachomatis
CT LAMP primer B3

SEQ ID NO # 7

TCGAGCAACCGCTGTGACGACCTTCATTATGTCGGAGTC
Chlamydia trachomatis
CT LAMP primer FIP

SEQ ID NO # 8

GCAGCTTGTAGTCCTGCTTGAGTCTTCGTAACTCGCTCC
Chlamydia trachomatis
CT LAMP primer BIP

SEQ ID NO # 9

TACAAACGCCTAGGGTGC
Chlamydia trachomatis
CT LAMP primer LF

SEQ ID NO # 10

CGGGCGATTTGCCTTAAC
Chlamydia trachomatis
CT LAMP primer LB

SEQ ID NO # 11

GCTCTGACTCCATTATCCTATG
Neisseria gonorrhoeae
NG LAMP primer 2086_LD39_F3

SEQ ID NO # 12

GCATTCAGACGTGCTTCTA
Neisseria gonorrhoeae
NG LAMP primer 2086_LD39_B3

SEQ ID NO # 13

TTGCCGCCTAAGGTTCCGAGCTATGGCATTAGGATTGTC
Neisseria gonorrhoeae
NG LAMP primer 2086_LD39_FIP

SEQ ID NO # 14

TGGAACGAGCCAAGGTGTTCTCTTGCATCAACTGTTCCAT
Neisseria gonorrhoeae
NG LAMP primer 2086_LD39_BIP

SEQ ID NO # 15

GCGGTCATTGACGAAATGG
Neisseria gonorrhoeae
NG LAMP primer 2086_LD39_LF

SEQ ID NO # 16

GGCTACAAGTGATAAGGTGCT
Neisseria gonorrhoeae
NG LAMP primer 2086_LD39_LB

SEQ ID NO # 17

AATCAAGAATACCGCCTCAC
Neisseria gonorrhoeae
NG LAMP primer 1430_LD31_F3

SEQ ID NO # 18

AGTTCTACGAACACTTGCTG
Neisseria gonorrhoeae

NG LAMP primer 1430_LD31_B3

SEQ ID NO # 19

TTGGAGATGTGGCGTTCGGACGACATCAACAACGACC
Neisseria gonorrhoeae
NG LAMP primer 1430_LD31_FIP

SEQ ID NO # 20

CGGAAGCAGGCGTCATCAGTTGTTTGGTCGCAAGGA
Neisseria gonorrhoeae
NG LAMP primer 1430_LD31_BIP

SEQ ID NO # 21

TGTCCAATGCGTCGTTGA
Neisseria gonorrhoeae
NG LAMP primer 1430_LD31_LF

SEQ ID NO # 22

CGCCATGCCGACAATTAC
Neisseria gonorrhoeae
NG LAMP primer 1430_LD31_LB

SEQ ID NO # 23

GACGTCAAGTCCTCATGG
Neisseria gonorrhoeae
NG LAMP primer 16S_id07_F3

SEQ ID NO # 24

CGGTTACCCTACCTACTTCT
Neisseria gonorrhoeae
NG LAMP primer 16S_id07_B3

SEQ ID NO # 25

TTGTGAGATTGGCTCCGCTCTCACACGTCATACAATGGTC
Neisseria gonorrhoeae
NG LAMP primer 16S_id07_FIP

SEQ ID NO # 26

TCGAGTGCATGAAGTCGGAATCGAACGTATTCACCGCAGT
Neisseria gonorrhoeae
NG LAMP primer 16S_id07_BIP

SEQ ID NO # 27

GCTTGGCTACCCTCTGTAC
Neisseria gonorrhoeae
NG LAMP primer 16S_id07_LF

SEQ ID NO # 28

CTAGTAATCGCAGGTCAGCAT
Neisseria gonorrhoeae
NG LAMP primer 16S_id07_LB

*Primer variation*

In addition to specific target regions, there are several key factors to design the appropriate primers for the amplification. The $T_m$ for each region is designed to be about 65°C (64 - 66°C) for F1c and B1c, about 60°C (59 - 61°C) for F2, B2, F3, and B3, and about 65°C (64 - 66°C) for the loop primers.

The end of the primers serves as the starting point of the DNA synthesis and thus must have certain degree of stability. The 3' ends of F2/B2, F3/B3, and LF/LB and the 5' end of F1c/B1c are designed so that the free energy is -4 kcal/ mol or less. The 5' end of F1c after amplification corresponds to the 3' end of F1, so that stability is important.

Primers are designed so that their GC content is between about 40% to 65%. It is important, particularly for the Inner primer, that primers are designed so that they do not form secondary structures.

The primers are designed so that the distance from the end of F2 to the end of B2 (the region amplified by the LAMP method) is between 120 bases and 160 bases. The primers are also designed so that the distance from the 5' end of F2 to the 5' end of F1 (the portion that forms the loop) is between 40 bases and 60 bases. The primers are also designed so that the distance between F2 and F3 is between 0 to 60 bases.

The primers are designed so that they do not form primer dimers that will result in drop of amplification efficiency and/or false positive signal in detection and visualization procedure.

In the current invention, the described target regions of primers are:

CDS2 region of Chlamydia trachomatis plasmid
Genomic region 16SrRNA and hypothetical protein regions 2086 and 1430 of Neisseria gonorrhoeae.

Thus, in one embodiment, the present invention relates to primers for the detection of sexually transmitted diseases such as but not limited to Chlamydia and/or Gonorrhoea in a sample, comprising: a nucleic acid molecule that encodes any of the nucleotide sequences selected from the group comprising SEQ ID NO: 5-28 or a part of it or a nucleotide having at least 90% sequence identity with said SEQ ID NO: 5-28.

*Detection step*

*Lateral flow strips*

Lateral flow strips are simple devices intended to detect the presence (or absence) of a target analyte in sample (matrix) without the need for specialized and costly equipment.

Typically, these tests are used for medical diagnostics either for home testing, point of care testing, or laboratory use. A widely spread and well known application is the home pregnancy test.

The technology is based on a series of capillary beds, such as pieces of porous paper or sintered polymer. Each of these elements has the capacity to transport fluid (e.g., urine) spontaneously. The first element (the sample pad) acts as a sponge and holds an excess of sample fluid. Once soaked, the fluid migrates to the second element (conjugate pad) in which the manufacturer has stored the so-called conjugate, a dried format of bio-active particles (see below) in a salt-sugar matrix that contains everything to guarantee an optimized chemical reaction between the target molecule and its chemical partner that has been immobilized on the particle's surface.

While the sample fluid dissolves the salt-sugar matrix, it also dissolves the particles and in one combined transport action the sample and conjugate mix while flowing through the porous structure.

In this way, the analyte binds to the particles while migrating further through the third capillary bed. This material has one or more areas (often called stripes) where a third molecule has been immobilized by the manufacturer. By the time the sample-conjugate mix reaches these strips, analyte has been bound on the particle and the third 'capture' molecule binds the complex. After a while, when more and more fluid has passed the stripes, particles accumulate and the stripe-area typically changes color.

Typically, there are at least two stripes: one (the control) that captures any particle and thereby shows that reaction conditions and technology worked fine, the second contains a specific capture molecule and only captures those particles onto which an analyte molecule has been immobilized. After passing these reaction zones the fluid enters the final porous material, the wick, that simply acts as a waste container. Lateral Flow Tests can operate as either competitive or sandwich assays.

In one embodiment, the LAMP reactions analysed in a lateral flow strip. These strips are used for qualitative signalling, when only "yes" or "no" answer is aimed. A lot of point-of-care rapid test devices are using lateral flow bases and plasmonic signal enhancement with e.g. gold nanoparticle, such as the are widely used pregnancy test, drug abuse test, allergy tests, etc.

Lateral flow strip based signal visualization and enhancement is very beneficial on cost perspective, this is presumably the cheapest solution to form simple optical readout that is applicable for point-of-care devices.

While using lateral flow strips for amplicon detection and signal visualization one should also bear in mind that upstream components, including those that are coming from sample matrix, will not inhibit the very binding reaction on lateral flow. This also usually requires balancing of lateral flow buffer and amount of conjugated gold nanoparticles that are required. Wrong balancing of components will result in signal loss or in nonspecific signals. Thus, providing a point of care with lateral flow strip based signal visualization

Different haptens can be used for the labeling of the primers, including FAM, biotin, DIG, in order to detect amplification product with lateral flow strips. Using different hapten combination multiple product detection can be achieved (multiplex reaction detecting several different pathogens at once).

*Assay time*

In one embodiment of the present invention the total assay time is less than 60 minutes. Assay turnaround time depends on balancing of assay enhancers and inhibitors. Particularly, it is important to balance out an optimum concentration of all components. Processes in nature follow dynamic principle, where too low concentrations will not have an effect and too high concentrations will supress and inhibit the entire process causing a failure of assays. Therefore, in the present invention components are balanced respectively, so that assay turnaround time should not exceed 60 min. This is user specified benchmark is taken from market analysis performed by the applicant, where users of such point-of-care devices have reported that they expect to have turnaround time less than 60 minutes, although longer assay times of course are possible.

In a presently preferred embodiment, the assay time may be split in the following steps:

Sample pretreatment time (with peptide) - typically 1-15 min, or at least sufficient time to ensure efficient lysis and nucleic acid release

Amplification time - typically 21-40 min; if lowered, no amplification will be detected at low template concentrations; if increased, there is a chance of getting a non-specific amplification signal (false positive)

Detection time - typically 2-5 min, depending on the lateral flow strip (false negative if LF signal is read too early, false positive if read too late)

As the skilled addressee would recognize, then the assay time can be varied and the above split is primarily to show the distribution between the individual steps. Depending on the used components, individual steps durations can be shorter or longer. Important is that the turnaround time of the entire assay should not exceed reasonable expected time in perspective point-of-care device user. In the present invention this is limited to preferably less than 60 minutes.

*Likelihood of infection*

The signal detected in the method of the present invention can be correlated with a predetermined value in order to indicate infection of the specific pathogen.

Such predetermined value can depend on many parameters including ROC curve characteristics, and values for specific settings depending on the dyes used or for example the bacteria to be detected.

In another embodiment of the present invention is the signal detected correlated with a standard in order to indicate the likelihood of infection and/or distinguish between acute or latent phase (chronic) of infection or an influence of consecutive infections as it is described in literature ("*Correlating Chlamydia trachomatis infectious load with urogenital ecological success and disease pathogenesis"*, João P. Gomes, Maria J. Borrego, Berna Atik, Irene Santo, Jacinta Azevedo, Armando Brito de Sá, Paulo Nogueira, Deborah Dean, *Microbes and Infection* 8 (2006) 16-26).

*Quantitative signal*

Quantitative signals indicate the amount, level or numbers of bacteria present in the sample. As in quantitative polymerase chain reaction, addition of e.g. fluorescent dyes enables quantitative real-time monitoring of reaction product formation in quantitative LAMP (qLAMP).

These types of signals can potentially be very important in order to evaluate the severity of the infection.

Thus, in one embodiment of the present invention, the method comprises a quantitative signal or an absolute signal for the presence of the nucleic acid.

*Absolute signal*

An absolute signal is an "on or off" signal that detects the presence of a nucleic acid of choice. Thus, any detection of an absolute signal indicates the presence of a pathogen.

These types of signals are usually subject to a standard, such as a predetermined value or a ROC curve, as discussed above.

*Sensitivity and specificity*

In one embodiment of the present invention, the sensitivity is higher than 70%.

In one embodiment, the sensitivity is calculated, when comparing to the Cobas®4800 CT/NG Test (Roche) in standard settings as they are stipulated in instruction manual.

Increase of sample volume used in assay or use of sample enrichment techniques allow to increase the sensitivity. Since NAAT methods are very specific, therefore the overall specificity is near to 100% whereas affected by chance to failure resulting in the range 95-100%.

The sensitivity of any given diagnostic test defines the proportion of individuals with a positive response who are correctly identified or diagnosed by the test, e.g. the sensitivity is 100%, if all individuals with a given condition have a positive test. The specificity of a given screening test reflects the proportion of individuals without the condition who are correctly identified or diagnosed by the test, e.g. 100 % specificity is, if all individuals without the condition have a negative test result.

Sensitivity is defined as the proportion of individuals with a given condition (e.g. active infection), who are correctly identified by the described methods of the invention (e.g. has a positive LAMP test result).

Specificity herein is defined as the proportion of individuals without the condition (e.g. active infection), who are correctly identified by the described methods of the invention (e.g. has a negative test result)

*Receiver-operating characteristics*

Accuracy of a diagnostic test is best described by its receiver-operating characteristics (ROC) (see especially Zweig, M. H., and Campbell, G., Clin. Chem. 39 (1993) 561-577). The ROC graph is a plot of all of the sensitivity/specificity pairs resulting from continuously varying the decision threshold over the entire range of data observed.

The clinical performance of a diagnostic test depends on its diagnostic accuracy, or the ability to correctly classify subjects into clinically relevant subgroups. Diagnostic accuracy measures the test's ability to correctly distinguish two different conditions of the subjects investigated. Such conditions are for example health and disease, latent or recent infection versus no infection, or benign versus malignant disease.

In each case, the ROC plot depicts the overlap between the two distributions by plotting the sensitivity versus 1 - specificity for the complete range of decision thresholds. On the y-axis is sensitivity, or the true-positive fraction [defined as (number of true-positive test results) (number of true-positive + number of false-negative test results]. This has also been referred to as positivity in the presence of a disease or condition. It is calculated solely from the affected subgroup. On the x axis is the false-positive fraction, or 1 - specificity [defined as (number of false- positive results) / (number of true-negative + number of false-positive results)]. It is an index of specificity and is calculated entirely from the unaffected subgroup.

Because the true-and false-positive fractions are calculated entirely separately, by using the test results from two different subgroups, the ROC plot is independent of the prevalence of disease in the sample. Each point on the ROC plot represents a sensitivity/- specificity pair corresponding to a particular decision threshold.

A test with perfect discrimination (no overlap in the two distributions of results) has an ROC plot that passes through the upper left corner, where the true- positive fraction is 1.0, or 100% (perfect sensitivity), and the false- positive fraction is 0 (perfect specificity). The theoretical plot for a test with no discrimination (identical distributions of results for the two groups) is a 45° diagonal line from the lower left corner to the upper right corner. Most plots fall in between these two extremes. (If the ROC plot falls completely below the 45° diagonal, this is easily remedied by reversing the criterion for "positivity" from "greater than" to "less than" or vice versa.) Qualitatively, the closer the plot is to the upper left corner, the higher the overall accuracy of the test.

One convenient goal to quantify the diagnostic accuracy of a laboratory test is to express its performance by a single number. The most common global measure is the area under the ROC plot. By convention, this area is always >_ 0.5 (if it is not, one can reverse the decision rule to make it so). Values range between 1.0 (perfect separation of the test values of the two groups) and 0.5 (no apparent distributional difference between the two groups of test values). The area does not depend only on a particular portion of the plot such as the point closest to the diagonal or the sensitivity at 90% specificity, but on the entire plot. This is a quantitative, descriptive expression of how close the ROC plot is to the perfect one (area = 1.0).

Clinical utility of the present invention may be assessed in comparison to and in combination with other diagnostic tools for the given infection.

In the case of infection with e.g. Chlamydia and/or Neisseria clinical utility of the present invention may be assessed in comparison to established diagnostic tools such as e.g. Cobas®4800 CT/NG Test (Roche) or other comparable gold standard PCR methods. See Example 1 and 6, where the inventive assays of the present invention on clinical samples is compared with the Cobas®4800 CT/NG Test as reference method, resulting in the given sensitivity values.

Thus, it is an object of preferred embodiments of the present invention to provide a method for detecting whether a human has encountered a sexually transmitted infectious pathogen, the method comprising:

a) determining the level of LAMP amplification product in a urine sample of said human,

b) constructing a percentile plot of the LAMP amplification product level obtained from a healthy population,

c) constructing a ROC (receiver operating characteristics) curve based on the LAMP amplification product level determined in the healthy population and on the LAMP amplification product level determined in a population who was diagnosed with the sexually transmitted infectious pathogen in question,

d) selecting a desired specificity,

e) determining from the ROC curve the sensitivity corresponding to the desired specificity,

f) determining from the percentile plot the LAMP amplification product level corresponding to the determined sensitivity; and

g) predicting the individual to have a sexually transmitted infectious pathogen, if the level of LAMP amplification product in the sample is equal to or higher than said LAMP amplification product level corresponding to the determined specificity and predicting the individual as unlikely or not to having a sexually transmitted infectious pathogen, if the level of LAMP amplification product in the sample is lower than said LAMP amplification product level corresponding to the determined specificity.

The specificity of the method according to the present invention may be from 70% to 100%, more preferably 80% to 100%, more preferably 90% to 100%. Thus in one embodiment of the present invention the specificity of the invention is 80%, 81%, 82%, 83%, 84%, 85%, 86%, 87%, 88%, 89%, 90%, 91%, 92%, 93%, 94%, 95%, 96%, 97%, 98%, 99% or 100%.

The sensitivity of the method according to the present invention may be from 60% to 100%, preferably 70% to 100%, more preferably 80% to 100%, even more preferably 90% to 100%.

Thus, in one embodiment of the present invention the sensitivity of the invention is 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%.

The level of LAMP amplification product is compared to a set of reference data or a reference value such as the cut-off value to determine whether the subject is at an increased risk or likelihood of e.g. infection.

To increase detection efficiency the LAMP amplification product may be compared to a set of reference data to determine whether the subject is likely to be infected or is at increased risk of developing of e.g. infection.

To increase detection efficiency, LAMP amplification product may be compared to a set of reference data to determine whether the subject has the infection or is at increased risk of developing infection or disease.

To determine whether the human is at increased risk of developing e.g. infection, a cut-off must be established. This cut-off may be established by the lateral flow strip, the physician or on a case by case basis by each subject.

Alternatively cut point can be determined as the mean, median or geometric mean of the negative control group ((e.g. not infected, healthy unexposed, patients without infection) +/- one or more standard deviations or a value derived from the standard deviation).

*Risk assessment*

Cut-off points can vary based on specific conditions of the individual tested such as but not limited to the risk of having the disease, occupation, geographic residence or exposure.

Cut-off points can vary based on specific conditions of the individual tested such as but not limited to age, sex, genetic background (i.e. HLA-type), acquired or inherited compromised immune function (e.g. HIV infection, diabetes, patients with renal or liver failure, patients in treatment with immune-modifying drugs such as but not limited to corticosteroids, chemotherapy, TNF-o blockers, mitosis inhibitors).

Doing adjustment of decision or cut-off limit will thus determine the test sensitivity for detecting an infection, if present, or its specificity for excluding infection or disease if below this limit. Then the principle is that a value above the cut-off point indicates an increased risk and a value below the cut-off point indicates a reduced risk.

*Cut-off levels*

As will be generally understood by those of skill in the art, the methods of the present invention are processes of decision making by comparison. For any decision-making process, reference-values based on subjects having the disease or condition of interest and/or subjects not having the disease, infection, or condition of interest are needed.

The cut-off level (or the cut-off point) can be based on several criteria including the number of subjects who would go on for further diagnostic testing, the average risk of having and/or developing e.g. infection to all the subjects who go on for further diagnostic testing, a decision that any subject whose patient specific risk is greater than a certain risk level such as e.g. 1 in 400 or 1:250 (as defined by the screening organization or the individual subject) should go on for further diagnostic testing or other criteria known to those skilled in the art.

The cut-off level can be adjusted based on several criteria such as but not restricted to certain group of individuals tested. E.g. the cut-off level could be set lower in individuals with immunodeficiency and in patients at great risk of progressing to active disease, cut-off may be higher in groups of otherwise healthy individuals with low risk of developing active disease.

The discriminating value is a value which has been determined by measuring the parameter or parameters in both a healthy control population and a population with known infection thereby determining the discriminating value which identifies the infected population with either a predetermined specificity or a predetermined sensitivity based on an analysis of the relation between the parameter values and the known clinical data of the healthy control population and the infection patient population, such as it is apparent from the detailed discussion in the examples herein.

The discriminating value determined in this manner is valid for the same experimental setup in future individual tests. Other experimental setups, other samples, other antigens, and other parameters will of course result in other cut-off values, which can be determined in accordance with the teachings herein by a person skilled in the art by normal design procedures or routine experiments.

*Cross reactivity*

Cross-reactivity with other pathogenic DNA is a huge threshold for POC diagnostics. At least 30 pathogen's DNA may potentially be present in a urine samples. Specific primers for detection of the pathogen of interest should not detect other species DNA to assure high specificity of the assay. The primers of the present invention is tested for sufficient sensitivity and specificity without having cross reactivity, which compromise the use in POC testing.

*Heat treatment*

In a further embodiment of the present invention, the antimicrobial peptide based release of nucleic acids is combined with a heat treatment.

Higher temperatures simply accelerate eukaryotic and prokaryotic cell lysis process thus, combined use enhances and lowers the time needed for nucleic acid release. Cellular membranes are efficiently degraded upon increase of the temperature, that facilitates release of the nucleic acids that are the target of the diagnostics assay.

*Use of surfactants*

In another embodiment of the present invention, the antimicrobial peptide based release of nucleic acids is combined with surfactants. The use of surfactants (such as Triton X-100, Triton X-114, NP-40, CHAPS, CTAB, Tween 20, Tween 80, Octyl beeta-thioglucoside, Octyl beeta-glucoside or others) is most useful for lysing intracellular parasites where they help to break host cells.

On Figure 4 is shown that the use of surfactant can slightly increase lysis efficiency.

*Kit of parts*

Kits for the detections of pathogens using the methods of the present inventions are highly relevant.

Such kit will comprise; a buffer comprising the antimicrobial drug targeting a bacterium of choice, and a buffer comprising primers targeting the same bacteria. These may be comprised in the same buffer, and can furthermore comprise dyes, polymerase, salts and any other components that are needed for the amplification and detection of bacteria.

In one embodiment, the kit is based on LAMP for the simultaneous detection and quantitation of sexual transmitted diseases such as but not limited to Chlamydia trachomatis and/or Neisseria gonorrhoeae, said kit comprises

a) a lysis mixture in the following ranges:

0.5 - 5μM AMP (Cecropin P1)
5 - 50mM EDTA
0.1 - 10% non-ionic surfactant

b) a mixture comprising nucleotide sequences that encodes a nucleotide sequence selected from the group comprising or any combinations of SEQ ID NO 5-28, or a part of it or a nucleotide having 90% sequence identity with any of SEQ ID NO: 5-28, and/or a polymerase capable of mediating a Loop-mediated isothermal amplification (LAMP), and

c) a device comprising a lateral flow strip.

*General*

As used herein, the terms "patient" and "subject" are used interchangeably to refer to a human.
The following figures and examples are provided below to illustrate the present invention. They are intended to be illustrative and are not to be construed as limiting in any way.
Throughout this application, various publications are referenced.

BRIEF DESCRIPTION OF THE FIGURES

*Figure 1*
Urine tolerance of LAMP. LAMP urine tolerance was tested in the presence of 0 %, 5 %, 10 %, 15 % and 20 % of pooled patient urine. Results were analysed quantitatively (A) and using LF strips (B). For quantitative analysis, remaining amplification time was calculated as an average time compared to 0 % urine reaction at standard DNA dilutions 107, 105 and 104 target copies per reaction. For LF strips detection, reaction was performed in the presence of 100 template copies in five parallels; two bands confirm the successful outcome of DNA amplification and a negative result are indicated by the presence of a single black band on the test strip.

*Figure 2*
Probit curve for C. trachomatis -LAMP assay LOD in water and pooled urine.

*Figure 3*
Lytic effect of heat (90°C 5 min) and antimicrobial peptides (50 μM) on E. coli cell integrity. Flow cytometry analysis was conducted with double-staining lysed cells with SYTO9/PI combination. Lysis reaction was carried out in 10% artificial urine conditions. AMP 6 was used as a negative control (no growth inhibition effect in experiments above).

*Figure 4*
Lytic effect of AMPs in combination with detergent Triton-X-100. E. coli lysis was analysed with flow cytometry. AMP and Triton-X-100 concentrations in lysis mixture were 50 μM and 2%, respectively. Note: AMP 5 was acquired from PepScan instead of using our self-purified batch. Thus, there is a difference in lysis efficiency of AMP 5 if compared to results shown before.

*Figure 5*
Urine tolerance was analyzed for Bst (A) and Bsm (B) DNA polymerases with qLAMP (at 63°C) in the presence of 0%, 5%, 10%, 15% or 20% of pooled patient urine (from 5 CT negative mails and 5 females) at 107-104 template DNA concentration. Urine tolerance results were expressed as a relative time to results.

*Figure 6*
Loop-mediated isothermal amplification directly from E. coli cell lysates. Isothermal amplification after pretreatment of urine spiked E. coli with different lysate preparation techniques. Lysozyme was used at 1 mg/ml concentration, polyethylene glycol p-(1,1,3,3-tetramethylbutyl)-phenyl ether at 1% and peptides at 50 $\mu$M concentration for the biological sample treatment. All sample pretreatments were performed for 5 min at RT°C (except for heat treatment that was incubated at 95°C).

*Figure 7*
Loop-mediated isothermal amplification activity in the presence of different lytic agents. Percentage of amplification activity in the presence of 0.1% polyethylene glycol p-(1,1,3,3-tetramethylbutyl)-phenyl ether, 0.1 mg/ml lysozyme (with or without 0.1% polyethylene glycol p-(1,1,3,3-tetramethylbutyl)-phenyl ether or 1mM EDTA) or alkaline lysis components.

*Figure 8*
Loop-mediated isothermal amplification activity in the presence of different lytic peptides. Percentage of amplification activity in the presence of 5 $\mu$M antimicrobial peptides.

*Figure 9*
Urine tolerance comparison of different polymerases (Bsm, GspM2.0, GspM, Bst, Bst2.0, GspSSD, GspSSD2.0, OmniAmp, Tin, SD) measured with estimated amplification time to results in minutes.

EXAMPLES

*Example 1 - Efficient and Rapid Loop-Mediated Isothermal Amplification Based Method for Chlamydia trachomatis Detection Directly from Urine*

*Introduction and Overview*

The lack of sensitive tests for C. trachomatis detection makes it difficult to diagnose Chlamydia infection efficiently directly from unprocessed patient's samples.

Here the inventors present a rapid and sensitive assay based on loop-mediated isothermal amplification method (LAMP), which can detect at least 5 C. trachomatis pathogens per reaction (25 cryptic plasmid copies) directly from urine samples within 21 min. Our clinical data showed that specificity of the assay is 100 % and sensitivity 73 %. Additionally, we demonstrate that our assay does not give any cross-reactivity with at least 30 pathogen's DNA potentially present in the urine samples. Furthermore, assay's novel approach does not require purification or extraction of DNA from clinical sample prior to amplification, so the need for specialized equipment is eliminated. This makes the whole procedure significantly less laborious, less time-consuming and consequently less expensive for early detection and identification of infectious disease. C. trachomatis specific LAMP assay is relatively simple to perform and could therefore be applied in numerous point-of-care settings.

Chlamydia trachomatis is a widespread sexually transmitted obligatory intracellular human pathogen. Most often, C. trachomatis is prevalent in adolescents and young adults of age 15-25 who have new or multiple sexual partners. The pathogen can also be passed from an infected mother to her baby during vaginal childbirth. In men Chlamydia trachomatis usually cause prostatitis and epididymitis and is mostly symptomatic causing a mild to moderate, clear to white urethral discharge, burning sensation during urination or dysuria. However, women with this infection often have minimal or no symptoms which leads to late diagnosis of the Chlamydia infection. Long-term effect of the C. trachomatis infection has been in turns associated with cervicitis, pelvic inflammatory disease, ectopic pregnancy, and acute or chronic pelvic pain.

Chlamydia has two life cycle stages: elementary body (EB) and reticulate body (RB), that are distinguishable by morphological and biological properties. The extracellular EBs are highly infectious, dispersal and analogue to spore. Once EB enters the epithelial cells it transforms into the metabolically active RBs that are able to replicate and accumulate within cytoplasmic inclusions of the cell. RBs are then converted into the more resistant EBs and are released from the cell.

Being an obligate intracellular pathogen makes it difficult to culture C. trachomatis in laboratory. Treatment of C. trachomatis infection depends not only on the site of the infection but also on the patient's age and on the case of complexity. So it is very important to identify C. trachomatis in the early stage of infection and start immediate treatment as soon as possible to prevent the development of further long-term complications and decrease the chance of getting other infections such as N. gonorrhoeae or Human immunodeficiency virus.

The diagnosis of sexually transmitted Chlamydia infection involves collection of the samples: regularly urethral swab or urine specimen from males and endocervical or vaginal swab from females. Successful screening and preventing further spread and complications of Chlamydia depends on the ability to diagnose infections accurately, rapidly and inexpensively. Modern laboratories still use traditional testing methods such as cell culture and antigen based detection. The cell culture method is highly specific but has very low sensitivity, is expensive, slow (the result could be obtained only after 3 days) and requires special sample collection, storage and transport. Immunological assays like the enzyme immunoassay and direct fluorescent antibody (DFA) assay have low sensitivity and specificity as a cell culture method which limits the use of these tests in diagnostic field. As an alternative to the traditional laboratory methods, nucleic acid amplification tests (NAATs) were developed allowing detection of pathogen-specific DNA or RNA sequences. These tests are significantly more sensitive for the screening and diagnosis of genital Chlamydia infection because they can detect as little as single nucleic acid copy of the target. There are several different nucleic-acid amplification based tests for C. trachomatis detection: Abbott RealTime CT/NG uses ligase chain reaction (LCR); Aptima COMBO uses transcription-mediated amplification (TMA); BD ProbeTec ET use strand displacement amplification (SDA); Xpert CT/NG and Cobas CT/NG tests use real-time polymerase chain reaction (PCR). Loop-mediated isothermal amplification (LAMP) stands out to be a novel, highly sensitive and specific diagnostic tool because of the ease of performing and capability to diagnose a negligible amount of pathogen genetic material within an hour. LAMP method has several advantages over widely used PCR. LAMP is carried out at a constant temperature (60-65°C) and does not require a thermal cycler. LAMP uses 4-6 different primers (2 inner, 2 outer and/or 2 additional loop primers) and a polymerase with a strand displacement activity to identify 6 distinct regions on the target gene sequence. This allows to generate an amplification product containing of single-stranded loop regions where primers can bind without template denaturation. The additions of loop primers significantly accelerate amplification, increasing sensitivity and reducing reaction time. The amount of DNA product at the end of the LAMP reaction is considerably higher that than in PCR and can be simply visualized using metal ion indicators like calcein or such DNA biding dyes as SYBR green, ethidium bromide, picogreen, propidium iodide, hydroxy naphthol blue.

Here the present inventors have developed C. trachomatis specific LAMP assay for the rapid and sensitive pathogen detection from human urine with minimal processing steps. Developed assay allows detection of C. trachomatis directly from urine sample, providing rapid, sensitive, diagnosis with minimal need for training. Clinical analysis showed up to 73 % sensitivity and 100 % specificity of the developed C. trachomatis specific LAMP assay. The assay requires 21 min and the product can be detected using lateral-flow (LF) strips which is very beneficial for application in the point-of-care (POC) settings.

*Materials and Methods*

*Design and Selection of the primers*

All C. trachomatis specific LAMP assay primer sets were designed to target highly conserved region of the C. trachomatis cryptic plasmid within coding sequence 2 - CDS2. LAMP primers were designed using LAMP Designer 1.10 software (PREMIER Biosoft USA) and screened for their high sensitivity towards DNA target and for the absence of the non-specific background on lateral flow strips. The sequence of the best performing primer set is shown in Supplementary Table 1. Primers FIP and LF were 5'labelled with biotin and BIP and LB primers with FAM to allow product detection on the lateral flow strips. Primers were obtained from Microsynth AG (Balgach, Switzerland).

*C. trachomatis ATCC DNA sample*

Commercially available C. trachomatis strain UW-36/Cx genomic DNA (ATCC®VR-886D™) was obtained from ATCC (American Culture Collection) and CDS2 target copy number was determined by qPCR (Applied Biosystems) using pGL3-CDS2 plasmid and following primers: Fw 5'- CTCCTTGGAGCATTGTCTGG- 3'and Rw 5'-CGGATGCGATGAACAGTTTG-3'

*LAMP reaction*

C. trachomatis specific LAMP reaction was carried out according to the protocol supplied by Eiken Chemical Co. Ltd with LAMP reaction total volume 50 µl. Bst polymerase was replaced with Bsm polymerase and 1.2 µl gDNA (ATCC)/ or 5 µl of pre-treated urine sample. All LAMP reactions were performed at 63°C for indicated time period and analysed on the lateral flow strips (AMODIA Bioservice GmbH) according to manufacturer's protocol. Reaction product was cut with PmlI restrictase (Thermo Fisher Scientific Inc. USA) to confirm product specificity.

For quantitative LAMP (qLAMP) EvaGreen (Biotium) fluorescent and ROX (Thermo Fisher Scientific Inc. USA) reference dyes were added to the reaction. Primers without Biotin/FAM labelling were used and product was detected using Applied Biosystems 7900HT Real-Time PCR System. A standard curve was constructed using serial dilutions of BglII linearized pGL3-CDS2 plasmid. qLAMP was performed at 63°C with 40 cycles, each 1 min long, and data reading after 30 sec.

*Pooled urine*

Urine samples from 5 men and 5 women, all C. trachomatis negative were mixed together in equal volumes. The samples further denoted as pooled urine was used in C. trachomatis specific LAMP assay for inhibition and assay sensitivity analyses.

*Sample pre-treatment*

Clinical patients' urine samples (30 µl) were incubated for 5 min together with 1 x lysis mix (SelfD Technologie GmbH, Leipzig, Germany), containing lytic peptide (12 % from final volume) or pre-heated at 90°C for 5 min and then cooled down on ice. 5 µl of each pre-treated urine sample were then applied for C. trachomatis specific LAMP amplification.

*Statistical analysis*

Statistical analyses of C. trachomatis specific LAMP assay's limit of detection were performed with XLSTAT, 95 % confidence interval (CI) was calculated using logistic regression model. Assay sensitivity and specificity was calculated using online tool from MedCalc confidence interval for 95 %.

*Clinical specimen collection and storage*

First-void morning urine samples were collected from 650 patients from 18 to 25 year old (316 females and 334 males) attending Sexual Health Clinique (Tartu, Estonia) from October 2013 to December 2014. Urine samples were self-collected into a clean polypropylene container (with average sample volume of 25-35 ml) without preservative and led by patients to the Sexual Health Clinique on the same day. Criteria for patient enrolment in the study were recent change of the sexual partner, multiple sexual partners, unprotected sexual intercourse, sexually transmitted infection of the partner or symptoms of the sexually transmitted disease (like increased/ abnormal vaginal discharge, urethral discharge, abdominal pain, painful urination, dysuria, itching and redness in the genital area), pregnancy or prophylactics.

Urine samples were tested by Cobas®4800 CT/NG Test (Roche) for the presence of C. trachomatis and N. gonorrhoeae according to manufacturer's instruction by the United Laboratories of the Tartu University Hospital regularly on the second day of the sample collection but no longer than 72 h after collection.

The 91 urine samples were tested by C. trachomatis specific LAMP assay within 6 h after collection (not more than 24 h from collection). 71 C. trachomatis positive samples were frozen at -20°C and tested separately. Approval for the study was obtained from the Research Ethics Committee of the University of Tartu. Confidentiality was addressed in the patient information sheet.

*Results*

*Development of C. trachomatis specific LAMP assay*

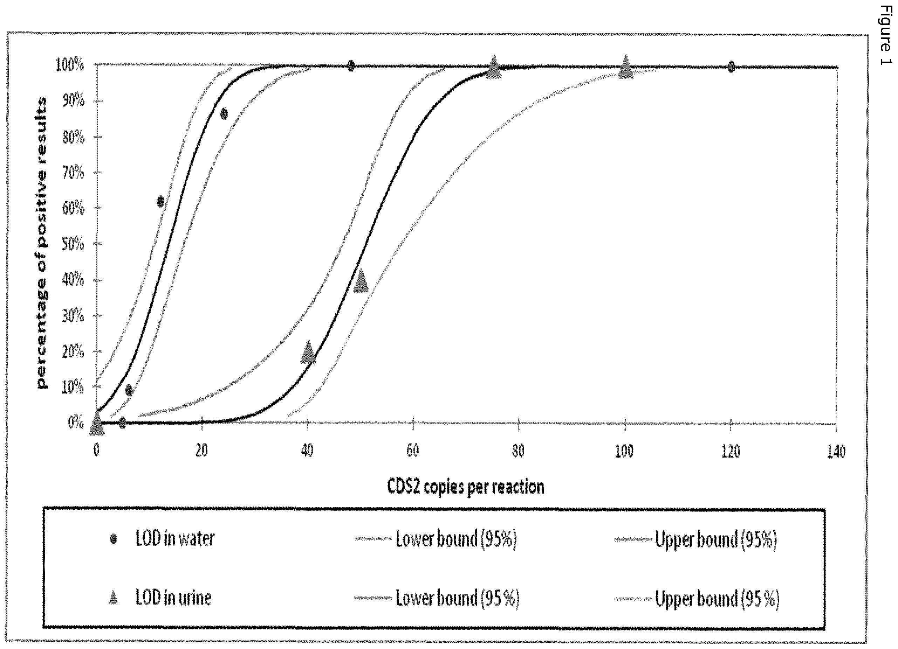

As the DNA purification step is not included in the LAMP assay, developed amplification method has to tolerate higher volumes of urine in the reaction mixture which theoretically can increase the whole assay sensitivity as more targets are added to the reaction. Therefore, urine tolerance of LAMP method was tested with pooled urine samples. In our experimental set-up LAMP reaction mixture contained up to 20 % of urine and 100 copies of template DNA. The result demonstrated that reactions containing 15 % of urine did not give any signal when analysed on lateral flow (LF) strips, indicating that 21 min of amplification time was insufficient for target amplification. 5-10 % of urine in reaction mixture gave positive signal in all replicates (n=5) (Figure 1A). This observation was also confirmed by qLAMP analysis (Figure 1B). Addition of 5-10 % urine did not affect amplification time. In the presence of 15-20 % of urine, the amplification time should be prolonged up to 30 min (Figure 1B). Therefore based on this data in all subsequent experiments 10 % of urine was used as a sample material in LAMP assay. In order to determine the minimal required amplification time, 100 template copies were used and reaction was terminated after 15 min, 18 min; 21 min; 24 min or 27 min. The result showed that minimum reaction time required for product formation on LF strips was 21 min (Supplementary Table 2). This enabled to reduce assay amplification procedure from recommended 1 h to 21 min without losing reaction sensitivity.

*Assessment of limit of detection (LOD) for C. trachomatis specific LAMP assay*

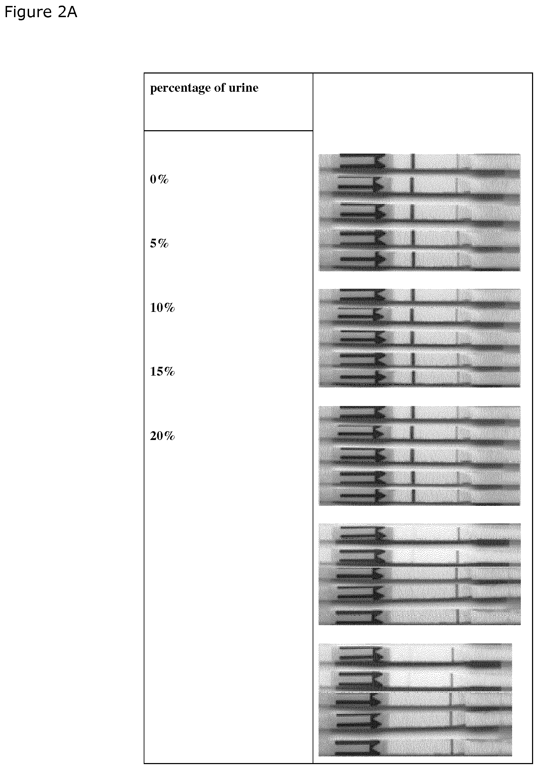

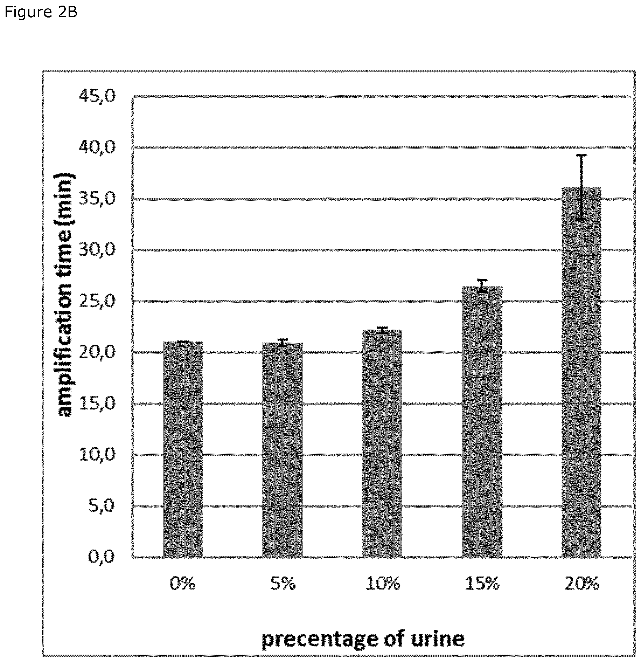

Next the lowest C. trachomatis plasmid copy number was determined by developed C. trachomatis specific LAMP assay. For this LAMP amplification efficiency was tested at different C. trachomatis genomic DNA concentrations in water or in 10 % pooled urine. Probit analysis showed that assay was sensitive enough to detect 25 plasmid copies per reaction in water (0.95 value) (Figure 2). As developed assay does not contain extra DNA purification step it was important to estimate if 10 % of crude urine can affect the sensitivity. For that pooled urine samples were spiked with different amount of C. trachomatis genomic DNA. As a result, in the presence of 10 % urine sensitivity of LAMP assay reduced approximately three times, and established LOD was 70 target copies per reaction (0.95 value) (Table 1). These results are in good correlation with urine inhibition experiment, where addition of 10 % urine prolonged LAMP amplification time (Figure 1) and can be also the reason why amplification activity was decreased approximately three times (Table 1).

*Table 1. LOD determination for C. trachomatis specific LAMP assay*

|  | Copies per reaction | Lower bound 95 % | Upper bound 95 % |
|---|---|---|---|
| **Limit of detection in water (0.95 value)** | 25 | 21 | 33 |
| **Limit of detection in pooled urine (0.95 value)** | 70 | 61 | 96 |

*Determination of C. trachomatis specific LAMP assay specificity*

Although C. trachomatis primers were designed targeting pathogen specific region, their specificity had to be experimentally verified. C. trachomatis LAMP assay was further analysed for possible non-specific reactivity using excess amount of genomic DNA from human and 30 microorganisms potentially present in human urine (listed in Supplementary Table S3). As a result C. trachomatis specific LAMP assay did not give cross-reactivity with any examined pathogens'DNA. Consequently, newly developed LAMP assay is highly specific in detection of Chlamydia trachomatis from human urine, fitting well for molecular diagnostic system.

*Clinical validation of the developed C. trachomatis specific LAMP assay*

To determine the prevalence of Chlamydia trachomatis in symptomatic and asymptomatic patients the urine samples subjected to clinical study (N= 650) were additionally evaluated. In order to analyse the collected data the descriptive statistics was used to provide important information received from patients and doctors. Patients included in the symptomatic group turned to the doctor for medical advice/control and had following symptoms: abnormal genital discharge, dysuria, abdominal pain, spotting between periods, itching or redness of the genital area. Patients, who had no symptoms, were included in the asymptomatic group. 157 out of 650 patients (24 %), had symptoms of sexually transmitted infection (STI) and 493 patients (76 %) were asymptomatic. 51 % of the patients were males and 49 % females, with an average age of 22 years old (18-25 years old). C. trachomatis was diagnosed in 39 male patients and 47 female patients out of which only 12 (31 %) and 13 (28 %) had symptoms, respectively. In all collected and analysed urine samples the prevalence of Neisseria gonorrhoeae was 3 %. Co-infection with N. gonorrhoeae /C. trachomatis seems to be quite frequent, 6-8 % from all C. trachomatis positive samples.

Clinical data confirmed that C. trachomatis infection is mainly asymptomatic, which makes it difficult to diagnose and estimate the real prevalence in human population (Table 2 and Supplementary Table 4).

Table 2. Clinical characteristics of the studied group (N=650)

| Gender | N (%) | Mean Age | SYM* | ASYM* | CT prevalence in the clinical study | SYM | ASYM | CT negative | SYM | ASYM |
|---|---|---|---|---|---|---|---|---|---|---|
| Male | 334 (51%) | 22 | 70 (21%) | 264 (79%) | 39 | 12 (31%) | 27 (69%) | 295 | 57 (19%) | 238 (81%) |
| Female | 316 (49%) | 22 | 87 (27.5%) | 229 (72.5%) | 47 | 13 (28%) | 34 (72%) | 269 | 74 (27.5%) | 195 (72.5%) |

* Patient info analysis presents gender distribution, number and percentage of the C. trachomatis (CT) positive patients with symptoms (SYM) and without symptoms (ASYM).

C. trachomatis specific LAMP assay clinical sensitivity and specificity was validated in a study, comprising 91 freshly collected patient urine samples. First-void morning urine samples were self-collected by 56 male and 34 female patients. 11 out of 91 had C. trachomatis infection (determined by Roche Cobas 4800 CT/NG test). One C. trachomatis positive patient was also positive for N. gonorrhoeae. Collected urine samples were subjected in parallel for C. trachomatis specific LAMP assay. Two different sample pre-treatment strategies were tested and compared to untreated samples: heating at 90°C for 5 min and incubation with peptide lysis mix. Results showed that highest assay sensitivity (73 %) was obtained when using peptide/detergent lysis mix whereas pre-treatment with heat showed 64 % of assay sensitivity, and sensitivity dropped to 55 % when urine samples were left untreated (Table 3). These data clearly shows that pre-treatment of urine samples prior to amplification provide a better access of pathogen DNA material.

Table 3. C. trachomatis detection in 91 first-void urine samples with the Roche Cobas Amplicor CT assay and C. trachomatis specific LAMP assay

| Fresh urine samples | | *C. trachomatis* LAMP assay | | | | | |
|---|---|---|---|---|---|---|---|
| | | Untreated urine* | | Heat treated urine** | | Peptide lysis mix pretreated urine*** | |
| | | Positive | Negative | Positive | Negative | Positive | Negative |
| Roche Cobas Amplicor CT assay | Positive | 6 | 5 | 7 | 4 | 8 | 3 |
| | Negative | 0 | 80 | 0 | 80 | 0 | 80 |
| Sensitivity % [95%CI] | | 55% [23,38-83,25%] | | 64% [30, 79- 89, 07%] | | 73% [39, 03- 93, 98%] | |
| Specificity % [95%CI] | | 100% [95, 5- 100%] | | 100% [95, 5- 100%] | | 100% [95, 5- 100%] | |

* 5 μl of fresh urine was added to LAMP reaction.** 5 μl of urine was incubated at 90°C for 5 min and after that added to LAMP reaction.*** 30 μl of urine was incubated with 4 μl (peptide lysis mix) for 5 min and after that 5 μl was taken out and added to LAMP reaction.

All 80 C. trachomatis negative samples were detected as negative by developed LAMP assay, thus clinical specificity was estimated to 100 %.

In order to confirm the results from the clinical study we analysed additional 71 C. trachomatis positive urine samples that were frozen at -20°C after collection. C. trachomatis specific LAMP assay was performed using the same pre-treated methods as described above. The sample set contained frozen C. trachomatis positive urines from 31 males and 40 females. Six C. trachomatis positive patients were also positive for N. gonorrhoeae and 9 patients had only N. gonorrhoeae infection. 48 out of 71 C. trachomatis positive urine samples tested positive with C. trachomatis specific LAMP assay using heat or peptide lysis mix. Analysis showed that both heating and lyses mix provided the sensitivity up to 68 % in frozen samples (Table 4). Storage of the urine sample in the freezer might be the reason why sensitivity was decreased from 73 % in "fresh", never frozen urine samples (urine lyses with peptide/detergent lysis mix) to 68 % (urine lyses with peptide/detergent lysis mix or heat) in frozen urine samples, because frozen urine yielded a big amount of urinary "microscopic sediments"compared to that found in "fresh"urine causing difficulties for sample pre-treatment. At the same time it can also be assumed that reaction temperature and freezing-thawing procedure itself can disrupt pathogen membrane, thereby releasing genomic material. That is why our assay showed 55 % ("fresh"urine) and 63 % (frozen urine) of sensitivity with untreated urine.

Table 4. Analysis of 71 frozen C. trachomatis positive urine samples with C. trachomatis specific LAMP assay

| Frozen urine samples | | *C. trachomatis* LAMP assay | | | | | |
|---|---|---|---|---|---|---|---|
| | | Untreated urine* | | Heated urine** | | Urine antimicrobial lyses*** | |
| | | Positive | Negative | Positive | Negative | Positive | Negative |
| Roche Cobas 4800 CT/NG assay | Positive | 45 | 26 | 48 | 23 | 48 | 23 |

(continued)

| Frozen urine samples | C. trachomatis LAMP assay | | | | | |
| --- | --- | --- | --- | --- | --- | --- |
| | Untreated urine* | | Heated urine** | | Urine antimicrobial lyses*** | |
| | Positive | Negative | Positive | Negative | Positive | Negative |
| Sensitivity % [95 % CI] | 63% [ 51.1- 74.5%] | | 68 % [55.5-78.24 %] | | 68 % [55.5-78.24 %] | |

*5 μl of frozen urine was added to the reaction mix.** 5 μl of frozen urine was incubated at 90°C for 5 min and then added to the reaction.*** 30 μl of frozen urine were incubated with 4 μl of 1 × lyses mix for 5 min out of which 5 μl was then added to reaction mix.

*Discussion*

Sexually transmitted infections (STIs), including Chlamydia trachomatis, remain an important focus area of the global public health. High number of cases within asymptomatic C. trachomatis infection (76 % in our clinical study) leads to delayed diagnosis and further serious complications. Although Chlamydia infection is highly prevalent, the knowledge regarding this STI was highly restricted in public health settings due to various limitations of diagnostic methods such as low pathogen detection sensitivity, low speed, time- consuming, expensiveness and need for specialist training. Consequently, successful detection and fast identification of this pathogen is necessary for early diagnosis and treatment of the disease.

This paper demonstrates for the first time, the development of simple, rapid, cost-effective, sensitive and specific LAMP assay for detection of C. trachomatis from urine samples. For that C. trachomatis cryptic plasmid has been chosen as a target for diagnostic testing because it is present at up to 10 copies per genome. Cryptic plasmid is relatively stable nucleic acid target being more resistant to nuclease damage than the genomic DNA. Chosen CDS2 region shares high homology between different C. trachomatis serovars and is present in the Swedish mutant strains. The use of six primers in LAMP reaction (F3, B3/ FIP, BIP/ LF, LB) provides not only a greater specificity than other amplification method but also accelerate the amplification time. Our data showed that novel C. trachomatis specific LAMP assay can detect at least 25 copies of the cryptic plasmid target which corresponds to 2.5-6 pathogens per reaction (depending on the particular strain). Consequently, the newly developed LAMP assay is 100 % specific in detection of Chlamydia trachomatis and can be easily applied in the point-of-care settings (POC). The whole assay takes less than 30 min, does not require additional equipment or trained personnel and the amplification product can be easily visualized by lateral flow dipsticks.

In focus of current research was also the detection of the pathogen with minimal processing steps directly from urine sample enabling simple and convenient application which is very advantageous for POC testing. As urine itself can contain various inhibitory substances it is quite challenging to use urine samples without prior DNA purification step before amplification. As it was already mentioned above our novel approach is based on loop-mediated amplification method (LAMP) and can detect pathogen directly from crude urine sample. To our knowledge only few reports related to detection of bacteria using LAMP method directly from biological sample are available so here we presented for the first time developed LAMP assay which enables detection of C. trachomatis directly in crude urine which thereby can replace current POC tests with pretty poor pathogen detection sensitivities (10- 40 %).

In spite of the fact that addition of 10 % urine slightly decreased analytical sensitivity (up to 3 times) the assay was still sensitive enough to detect Chlamydia from minimally processed patient samples. As human urine can contain different number of Chlamydia cells it was very important to successfully lyse pathogen cell membrane in order to better access the DNA material from the cells. For that different methods for cellular membrane lysis were tested. Heating at 90°C and the application of lytic peptides carry a potential to be applied for urine lysate preparation prior to nucleic acid amplification. Therefore our novel approach successfully combined LAMP amplification with fast sample preparation techniques: thermal or peptide lysis applying them directly in clinical samples. As a result the overall sensitivity of the novel C. trachomatis specific LAMP assay increased up to 73 % when using antimicrobial peptide lysis mix and 68 % when samples were heat pre-treated prior addition into the amplification reaction. As simple and easy to apply sample pre-treatment method is one of the major criteria for the true self-diagnostic tests it is very important to find the best strategy for lysing such challenging pathogen as Chlamydia directly in crude urine releasing its genetic material in such a way, that it would be suitable for the subsequent rapid amplification by LAMP method. Our data shows that the application of antimicrobial peptide lysis mix provides the most efficient newly developed sample pre-treatment strategy that suits all above mentioned requirements. Therefore there is no need to prior DNA purification and concentration, like in PCR-based Chlamydia detection assays, which makes the whole detection less laborious and time consuming. Such features as increased sensitivity, controllability, simplicity, rapid response time and high specificity of pathogen detection compared to other isothermal amplification methods or POC immunoassays make our novel C. trachomatis specific assay appealing for the application as the most reliable molecular diagnostic tool for POC testing. Taken together we have developed for the first time simple, rapid and sensitive LAMP assay based method for the specific detection of C. trachomatis directly from crude urine samples that have a great potential for the application in numerous POC settings.

*Supplementary Material*

*Table S1. LAMP primer set selected for C. trachomatis specific detection*

| Name | Sequence |
|------|----------|
| **F3** | 5' AAT ATC ATC TTT GCG GTT GC 3' |
| **B3** | 5' TCT ACA AGA GTA CAT CGG TCA 3' |
| **FIP** | 5' Biotin-TCG AGC AAC CGC TGT GAC GAC CTT CAT TAT GTC GGA GTC 3' |
| **BIP** | 5' FAM-GCA GCT TGT AGT CCT GCT TGA GTC TTC GTA ACT CGC TCC 3' |
| **LF** | 5' Biotin-TAC AAA CGC CTA GGG TGC 3' |
| **LB** | 5' FAM-CGG GCG ATT TGC CTT AAC 3' |

*Table S2. C. trachomatis specific LAMP assay amplification time*

| Reaction time (min) | Determination of percentage of positives* |
|---------------------|-------------------------------------------|
| 15 | 20% |
| 18 | 60% |
| 21 | 100% |
| 24 | 100% |
| 27 | 100% |

*Table S3. List of organisms used to determine specificity of the C. trachomatis LAMP assay*

| Species | Amount of DNA used per reaction |
|---------|---------------------------------|
| **Mammalian** | |
| Homo sapiens sapiens | 1 ng |
| **Protozoa** | |
| Trichomonas vaginalis | 1 ng |
| **Fungi** | |
| Candida albicans, | 1 ng |
| Saccaharomyces cerevisiae | 1 ng |
| **Bacteria** | |
| *Escherichia coli* | 24 pg, 100 pg |
| *Neisserea gonorrhoeae* | 24 pg, 100 pg |
| *Mycoplasma genitalium* | 24 pg, 100 pg |
| *Mycoplasma pneumoniae* | 24 pg, 100 pg |
| *Ureaplasma urealyticum* | 24 pg, 100 pg |
| *Mycoplasma hominis* | 24 pg, 100 pg |
| *Lactobacillus iners* | 24 pg, 100 pg |
| *Lactobacillus crispatus* | 24 pg, 100 pg |
| *Corynebacterium urealyticum* | 24 pg, 100 pg |

(continued)

| Bacteria | |
|---|---|
| *Prevotella bivia* | 24 pg, 100 pg |
| *Gardnerella vaginalis* | 24 pg, 100 pg |
| *Veillonella parvula* | 24 pg, 100 pg |
| *Enterococcus faecalis* | 24 pg,- 100 pg |
| *Proteus mirabilis* | 24 pg, 100 pg |
| *Klebsiella pneumoniae* | 24 pg, 100 pg |
| *Klebsiella oxytoca* | 24 pg, 100 pg |
| *Enterobacter cloacae subsp. cloacae* | 24 pg, 100 pg |
| *Citrobacter freundii,* | 24 pg, 100 pg |
| *Staphylococcus saprophyticus subsp. saprophyticus* | 24 pg, 100 pg |
| *Streptococcus agalactiae* | 24 pg, 100 pg |
| *Staphylococcus epidermidis* | 24 pg, 100 pg |
| *Staphylococcus aureus subsp. aureus* | 24 pg, 100 pg |
| **Viruses** | |
| *Herpes simplex 1* | 24 pg, 100 pg |
| *Herpes simplex 2* | 24 pg, 100 pg |
| *Human papilloma virus 16* | 24 pg, 100 pg |
| *Human papilloma virus 18* | 24 pg, 100 pg |

*Table S4. Data for the patients with co-infection with NG*

| Gender | CT&NG "+" | SYM | ASYM | CT"-"&NG "+" | SYM | ASYM |
|---|---|---|---|---|---|---|
| **Male** | 4 | 3 | 1 | 3 | 0 | 3 |
| **Female** | 3 | 3 | 0 | 6 | 1 | 5 |
| * Gender and number of C. trachomatis and N. gonorrhoeae co-infected patients (CT&NG "+"), and C. trachomatis negative and N. gonorrhoeae infected patients (CT"-"&NG "+") with symptoms (SYM) and without (ASYM). | | | | | | |

*Example 2 - Fast and highly efficient detection of Escherichia coli directly from urine samples using loop-mediated isothermal amplification.*

A biological sample containing E. coli bacteria (urine sample in Figure 6) is treated with 50μM antimicrobial peptide belonging to but not limited to cecropin group (Cecropin P1 or SB-37 in Figure 6) for 5 min at RT°C. 5μl of the urine sample is added directly to the loop-mediated amplification reaction, so that final reaction volume is 50 μl and final components concentration as follows: 0.2 μM F3 primer, 0.2 μM B3 primer, 1.6 μM BIP primer, 1.6 μM FIP primer, 0.8 μM LF primer, 0.8 μM LB primer, 8 mM MgSO4, 1.4mM dNTP, 0.8M betain, 20 mM Tris-HCl pH 8.8, 10 mM KCl, 10 mM (NH4)2SO4, 0.1% (v/v) Tween 20 and 16 units of Bsm polymerase.

EvaGreen and ROX dyes can be added to the LAMP reaction for quantitative product detection or FAM labeled BIP and LB primers and biotin labeled FIP and LF primers can be used for qualitative detection on lateral-flow strips.

In the example of E. coli detection from Figure 6, pretreatment of the urine samples with antimicrobial peptides gave up to 6 times increased amount of target DNA. Previously known and widely used thermal and alkaline sample preparation methods gained only in 3 and 2 times increase on the target DNA level (Figure 6). Thus new sample pretreatment method is well suitable with downstream amplification applications. Several detection methods can be used, both isothermal (like LAMP - loop-mediated isothermal amplification, SDA - strand displacement amplification, HDA - helicase-dependent amplification, RPA - recombinase polymerase amplification, TMA - transcription-mediated amplification, MDA - multiple displacement amplification, NASBA - nucleic acid sequence based amplification, NEAR - nicking enzyme amplification reaction) and the ones requiring thermocycling (like PCR, real-time PCR and others).

*Example 3 - Fast and sensitive detection of Mycoplasma genitalium directly from patient urine samples using recombinase polymerase amplification*

First-void morning urine sample is treated with 50$\mu$M antimicrobial peptide for 5 min at RT°C. For M. genitalium sample pretreatment melittin, gramicidin peptides or other antimicrobial peptides could be used that affect intracellular Mycoplasma species.

Antibacterial peptide concentration can be varied from low $\mu$M (0.1-1 $\mu$M) to high $\mu$M (100-1000 $\mu$M) depending on the peptide and biological sample type. Pre-treatment time can be increased or lowered depending on the peptide concentration, peptide type, target organism, biological sample type.

Biological sample can be concentrated or diluted prior to peptide treatment, if required. To stimulate sample pretreatment method detergents, lytic enzymes, alkali or other additional compositions can be used, if required.

The RPA enzyme pellet is resuspended with 47.5 $\mu$l of the reaction buffer prepared by mixing the following components: 2.1 $\mu$l of 10 mM forward primer, 2.1 $\mu$l of 10 mM reverse primer (the final concentration of each primer in the reaction being 0.42 mM), 29.5 $\mu$l of rehydration buffer (TwistDX), 8.8 $\mu$l of ddH2O) and 5$\mu$l of the biological sample lysate (prepared as described above) potentially containing M. genitalium. RPA reaction is initiated by adding 2.5 $\mu$l of 280 mM magnesium acetate.

M. genitalium specific primers targeting MGPA and 16S rRNA conserved unique regions can be used, examples of appropriate sequences are listed in patent application no PCT/EP2013/071906. Forward primers are labeled with biotin and the reverse primer with 6-carboxyfluorescein (FAM), enabling biotin-FAM double-labeled product detection on the lateral-flow strips. Different oligonucleotide labeling can be used, like DIG or other oligonucleotide linkers.

The reaction is incubated at 38°C for 10-30 minutes. After 4 minutes of incubation, the reaction was mixed by flicking the tube. The whole procedure takes 15-35 min, to obtain qualitative lateral-flow strip result of M. genitalium infection.

*Example 4 - Fast and sensitive detection of Neisseria gonorrhoeae directly from patient urine samples using loop-mediated isothermal amplification*

A biological sample including urine sample, vaginal, rectal, cervical or urethral swab is collected. First-void morning sample is used in case of urine to ensure presence of sufficient number of pathogen nucleic acid. Biological sample can be concentrated or diluted prior to peptide pre-treatment, if required. Sample is treated with 50$\mu$M antimicrobial peptide for 5 min at RT°C. For Neisseria species following antimicrobial peptides could be used, but not limited to demaseptins, LL-37, PG-1. Antibacterial peptide concentration can be varied and detergent (or other composition) could be added to stimulate lytic effect of the peptide. Pre-treatment time can be increased or lowered depending on the peptide concentration and type of the biological sample.

5$\mu$l of the biological sample lysate is added directly to the loop-mediated amplification reaction, so that final reaction volume is 50 $\mu$l and final components concentration as follows: 0.2 $\mu$M F3 primer, 0.2 $\mu$M B3 primer, 1.6 $\mu$M biotin labeled BIP primer, 1.6 $\mu$M FAM labeled FIP primer, 0.8 $\mu$M FAM labeled LF primer, 0.8 $\mu$M biotin labeled LB primer, 8 mM MgSO4, 1.4mM dNTP, 0.8M betain, 20 mM Tris-HCl pH 8.8, 10 mM KCl, 10 mM (NH4)2SO4, 0.1% (v/v) Tween 20 and 16 units of Bsm polymerase.

Amount of biological sample per amplification reaction can be varied depending on the sample type and required sensitivity. MgSO4, dNTP and betain concentrations can be varied to obtain optimal sensitivity depending on the specific target and polymerase used in the reaction. Different oligonucleotide labeling can be used, like DIG or other oligonucleotide linkers. For N. gonorrhoeae specific and sensitive detection primer sequences listed in Table 8 can be used targeting Opa, PorA, pilin or 16S rRNA genes.

After 20-25 min incubation at 63°C, N. gonorrhoeae specific product formation is analyzed with lateral-flow strips. If patient is N. gonorrhoeae positive, two stripes appear on the lateral-flow strip, one in the "test" area and another in the "control" area. If the patient is N. gonorrhoeae negative, only one stripe appears on the lateral-flow strip in the "control" area. The completely N. gonorrhoeae testing procedure takes up to 30 min.

*Example 5 - Effect of antimicrobial peptides on the downstream amplification efficiency.*

The described method does not require removal of the lysate preparation compositions prior to amplification, thus the effect of the selected membrane active peptides should be assessed on the downstream amplification.

Several efficient sample preparation methods contain components like SDS or Proteinase K that are highly inhibitory for the downstream amplification reaction, and therefore are not applicable for direct amplification from biological sample lysates but require additional step of purification of the nucleic acids.

To estimate potential inhibitory effect of different lysate preparation methods (alkaline, enzymatic, detergent lysis) and of antimicrobial peptides on downstream application efficiency, we have performed LAMP isothermal amplification in the presence of different lytic agents (Figure 4-5).

Addition of the lysozyme to the LAMP reaction had a very modest effect on the amplification efficiency. However, in combination with lysis enhancing agents (polyethylene glycol p-(1,1,3,3-tetramethylbutyl)-phenyl ether or EDTA), isothermal amplification efficiency was significantly reduced. Alkaline lysis components had a major effect on the isothermal amplification, reducing LAMP activity up to 25 times (Figure 4). Thus, several lysis preparation methods' composition inhibit significantly downstream application limiting their application.

Antimicrobial peptides are often forming cationic amphiphilic structure to interact with the negatively charged lipid bilayer. $\alpha$-helical, $\beta$-turn, antiparallel $\beta$-sheet and $\beta$-hairpin structures are frequently found in natural DNA/RNA binding proteins. Thus antimicrobial proteins carry the potential to interfere with amplification through binding of the template DNA. Addition of magainin analogues MSI-78 and MSI-594 had indeed quite severe inhibiting effect on the amplification efficiency (Figure 5). Cecropins (P1 and SB-37) and Bombolitin III on the other hand displayed only mild inhibition of the LAMP reaction, while melittin had an inhibiting effect comparable to alkaline lysis components (Figure 5). Thus, certain lytic peptides could be better suited for lysate preparation than others due to their effect on the downstream application. However taking into consideration cell lytic activity of the AMPs, their effect on the downstream amplification is quite modest, as compared to other lysate preparation methods.

*Example 6 - Inhibitory data*

Some samples may contain inhibitory components that will cause a failure of assay, specifically amplification reaction resulting in a false negative diagnosis results and thus influencing sensitivity of the assay. Current invention contains balanced sample pre-treatment and lysis components cocktail that also serves anti-inhibitory behaviour. Using this developed lysis mixture (cocktail) that consists in urine (treated):

0.1 $\mu$M AMP (Cecropin P1)
10 mM EDTA
0.4 % TX100 (surfactant)

in comparison of untreated urine samples shows as well a significant suppressing of inhibitory effect while the samples were spiked with CT (Chlamydia trachomatis) DNA, particularly 13% of samples. Thus within pre-treatment procedure is not only important a lysis of hosts and pathogen cells, it is also important to suppress inhibitory effects caused by sample matrix and its high variability among different patients.

| | untreated urine + CT-DNA | treated urine + CT-DNA |
|---|---|---|
| True positive | 33 | 38 |
| False negative | 5 | 0 |
| **Inhibition in %** | **13** | **0** |

*Example 7- Neisseria gonorrhoeae cross reactivity*

The present inventors have developed several primer sets for NG detection while using the same method as described for example 1 for Chlamydia trachomatis. The presently preferred ones are listed below. The set LOD (limit of detection) is 230 copies/rxn (per reaction). Also a cross reactivity (important to evaluate a chance for false positives) against other Neisseria strains was evaluated and results are as follows:

Cross-reactivity (focus to Neisseria strains)

| Primer | N. spp. | copies/rxn | Cross reactivity |
|--------|---------|-----------|------------------|
| 2086 | N. cinerea | 50,000 | - |
| | N. flava | | - |
| | N. meningitidis (serogroup B) | | - |
| | N. meningitidis (serogroup Y) | | - |
| | N. lactamica | | + |
| | N. perflava | | - |

Cross reactivity is only for *N. lactamica* that is actually rare strain to be found in urine

The very same cross reactivity was assessed with other primer sets as well and they showed higher cross reactivity rate, results are following:

| Primer | N.spp. | copies/rxn | Cross reactivity |
|--------|--------|-----------|------------------|
| 1430 | N. cinerea | 50,000 | + |
| | N. flava | | + |
| | N. meningitidis (serogroup B) | | - |
| | N. meningitidis (serogroup Y) | | - |
| | N. lactamica | | + |
| | N. perflava | | + |

Crossreactivity with *N. cinereal, N. flava, N. lactamica, N.*

| Primer | N.spp. | copies/rxn | Cross reactivity |
|--------|--------|-----------|------------------|
| 16S rRNA id07 | N. cinerea | 50,000 | + |
| | N. flava | | + |
| | N. meningitidis (serogroup B) | | + |
| | N.meningitidis (serogroup Y) | | + |
| | N. lactamica | | + |
| | N. perflava | | + |

Although this primer set showed the best LOD (77 copies/rxn) it has cross reactivity with all tested Neisseria-strains and thus not very suitable for diagnostic purposes.

Table 1. Primers to detect NG hypothetical protein 2086 target

| NG_hypothetical protein 2086 | 5' -> 3' |
|------------------------------|----------|
| 2086_LD39_F3 | GCTCTGACTCCATTATCCTATG |
| 2086_LD39_B3 | GCATTCAGACGTGCTTCTA |
| 2086_LD39_FIP | TTGCCGCCTAAGGTTCCGAGCTATGGCATTAGGATTGTC |
| 2086_LD39_BIP | TGGAACGAGCCAAGGTGTTCTCTTGCATCAACTGTTCCAT |
| 2086_LD39_LF | GCGGTCATTGACGAAATGG |

(continued)

| NG_hypothetical protein 2086 | 5' -> 3' |
|---|---|
| 2086_LD39_LB | GGCTACAAGTGATAAGGTGCT |

Table 2. Primers to detect NG hypothetical protein 1430_LD31 target

| NG-hypothetical protein 1430_LD31 | 5' -> 3' |
|---|---|
| 1430_LD31_F3 | AATCAAGAATACCGCCTCAC |
| 1430_LD31_B3 | AGTTCTACGAACACTTGCTG |
| 1430_LD31_FIP | TTGGAGATGTGGCGTTCGGACGACATCAACAACGACC |
| 1430_LD31_BIP | CGGAAGCAGGCGTCATCAGTTGTTTGGTCGCAAGGA |
| 1430_LD31_LF | TGTCCAATGCGTCGTTGA |
| 1430_LD31_LB | CGCCATGCCGACAATTAC |

Table 3. Primers to detect NG 16S rRNA id07 target.

| NG-16S rRNA id07 | 5' -> 3' |
|---|---|
| 165_id07_F3 | GACGTCAAGTCCTCATGG |
| 165_id07_B3 | CGGTTACCCTACCTACTTCT |
| 165_id07_FIP | TTGTGAGATTGGCTCCGCTCTCACACGTCATACAATGGTC |
| 16S_id07_BIP | TCGAGTGCATGAAGTCGGAATCGAACGTATTCACCGCAGT |
| 16S_id07_LF | GCTTGGCTACCCTCTGTAC |
| 16S_id07_LB | CTAGTAATCGCAGGTCAGCAT |

Cross reactivity is only for *N. lactamica* that is actually rare strain to be found in urine

The very same cross reactivity was assessed with other primer sets as well and they showed higher cross reactivity rate, results are following:

- Using the very same lysis mixture (that is 0.1 $\mu$M AMP/10 mM EDTA/0.4 % TX100 in urine) for NG detection, the sensitivity and the specificity is following:

| | *Neisseria gonorrhoeae* |
|---|---|
| Specificity in % | **100 %**   with 43 true negative urine samples |
| Sensitivity in % | **83.3 %**  with 12 true positive urine samples |

Using the very same lysis mixture (that is 0.1 µM AMP/10 mM EDTA/0.4 % TX100 in urine) for NG detection, the sensitivity and the specificity is following:

| | *Neisseria gonorrhoeae* |
|---|---|
| Specificity in % | **100 %**   with 43 true negative urine samples |
| Sensitivity in % | **83.3 %**  with 12 true positive urine samples |

**Claims**

1. A method for the detection of a sexually transmitted infectious pathogen in a human subject without sample purification, the method comprising the steps;

   a) providing a urine sample from the human subject,
   b) adding a cecropin peptide to the urine sample,
   c) amplifying the released nucleic acids by loop-mediated isothermal amplification (LAMP) using primers targeting the sexually transmitted infection pathogen nucleic acids,
   d) detecting a signal from the nucleic acid originating from the sexually transmitted infection pathogen organism, and
   e) indicating the human subject being infected with a sexually transmitted infectious pathogen, if the signal is above a predetermined value.

2. A method according to claim 1, wherein the urine sample is diluted more than 50% with a buffer.

3. A method according to any of claims 1-2, wherein the total assay time is less than 60 minutes.

4. A method according to any of claims 1-3, wherein the antimicrobial peptide-based release of nucleic acids is combined with a heat treatment.

5. A method according to any of claims 1-4, wherein the antimicrobial peptide-based release of nucleic acids is combined with surfactant(s).

6. A method according to any of claims 1-5, wherein LAMP reactions are analysed in a lateral flow strip.

7. A method according to any of claims 1-6, wherein the sexually transmitted infection pathogen primers are designed to detect Chlamydia trachomatis specific nucleic acids.

8. A method according to any of claims 1-7, wherein the sensitivity is higher than 60%.

9. A method according to any of claims 1-8, wherein the loop-mediated isothermal amplification (LAMP) comprise a Bsm polymerase.

10. A point-of-care method for the detection of Chlamydia trachomatis and/or Neisseria gonorrhoeae in a human subject without sample purification, the method comprising the steps;

    a) providing a urine sample diluted more than 50% from the human subject,
    b) adding a Cecropin peptide to the urine sample,
    c) amplifying the released nucleic acids by loop-mediated isothermal amplification (LAMP) using primers targeting Chlamydia trachomatis and/or Neisseria gonorrhoeae nucleic acids using a Bsm polymerase,
    d) detecting a signal from the nucleic acid originating from Chlamydia trachomatis and/or Neisseria gonorrhoeae by a lateral flow strip, and
    e) indicating the human subject being infected with Chlamydia trachomatis and/or Neisseria gonorrhoeae, if the signal is above a predetermined value,

wherein said point-of-care method have a detection sensitivity of more than 60% compared to the Cobas®4800 CT/NG Test (Roche) in standard settings.

11. A point-of-care method for the detection of Chlamydia trachomatis and/or Neisseria gonorrhoeae in a human subject without sample purification, the method comprising the steps;

    a) providing a urine sample,
    b) adding a Cecropin peptide to the urine sample, and allowing the peptide to lysis the cells for less than 15 minutes,
    c) diluting the urine sample obtained in step b) more than 50%
    c) amplifying the released nucleic acids by loop-mediated isothermal amplification (LAMP) using primers targeting Chlamydia trachomatis and/or Neisseria gonorrhoeae nucleic acids using a Bsm polymerase,
    d) detecting a signal from the nucleic acid originating from Chlamydia trachomatis and/or Neisseria gonorrhoeae by a lateral flow strip, and
    e) indicating the human subject being infected with Chlamydia trachomatis and/or Neisseria gonorrhoeae, if the signal is above a predetermined value,

wherein said point-of-care method has a detection sensitivity of more than 60% compared to the Cobas®4800 CT/NG Test (Roche) in standard settings.

12. A method according to any of the claims 1-11, wherein the antimicrobial peptide based release of nucleic acids is combined with surfactants.

13. A method according to any of claims 1-12, wherein the primers are selected from the group consisting of SEQ ID NO 5-28.

14. A kit of part for simultaneous detection and quantitation of sexual transmitted diseases such as but not limited to Chlamydia trachomatis and/or Neisseria gonorrhoeae, said kit comprises

    a) a lysis mixture in the following ranges:

        0.5 - 5$\mu$M AMP (Cecropin P1)
        5 - 50mM EDTA
        0.1 - 10% non-ionic surfactant

    b) a mixture comprising nucleotide sequences that encode a nucleotide sequence selected from the group comprising any combinations of SEQ ID NO 5-28, or a nucleotide having 90% sequence identity with any of SEQ ID NO: 5-28, and optionally a polymerase capable of mediating a Loop-mediated isothermal amplification (LAMP), and
    c) a device comprising a lateral flow strip.

**Patentansprüche**

1. Verfahren zum Nachweis eines sexuell übertragbaren infektiösen Krankheitserregers bei einem menschlichen Subjekt ohne Probenreinigung, wobei das Verfahren die folgenden Schritte umfasst;

   a) Bereitstellen einer Urinprobe des menschlichen Subjekts,
   b) Zusetzen eines Cecropin-Peptids zu der Urinprobe,
   c) Amplifizieren der freigesetzten Nukleinsäuren durch Loop-mediated Isothermal Amplification (LAMP) unter Verwendung von Primern, die auf die Nukleinsäuren des sexuell übertragbaren infektiösen Krankheitserregers abzielen,
   d) Nachweisen eines Signals von der Nukleinsäure, die von dem Organismus des sexuell übertragbaren infektiösen Krankheitserregers stammt und
   e) Anzeigen, dass das menschliche Subjekt mit einem sexuell übertragbaren infektiösen Krankheitserreger infiziert ist, wenn das Signal über einem vorbestimmten Wert liegt.

2. Verfahren nach Anspruch 1, wobei die Urinprobe zu mehr als 50 % mit einem Puffer verdünnt wird.

3. Verfahren nach einem der Ansprüche 1-2, wobei die Gesamtassay-Zeit weniger als 60 Minuten beträgt.

4. Verfahren nach einem der Ansprüche 1-3, wobei die auf antimikrobiellem Peptid basierte Freisetzung von Nukleinsäuren mit einer Wärmebehandlung kombiniert wird.

5. Verfahren nach einem der Ansprüche 1-4, wobei die auf antimikrobiellem Peptid basierte Freisetzung von Nukleinsäuren mit Tensid(en) kombiniert wird.

6. Verfahren nach einem der Ansprüche 1-5, wobei LAMP-Reaktionen in einem Lateral-Flow-Strip analysiert werden.

7. Verfahren nach einem der Ansprüche 1-6, wobei die Primer des sexuell übertragbaren infektiösen Krankheitserregers zum Nachweisen von Chlamydia trachomatis-spezifischen Nukleinsäuren ausgestaltet sind.

8. Verfahren nach einem der Ansprüche 1-7, wobei die Empfindlichkeit höher als 60 % ist.

9. Verfahren nach einem der Ansprüche 1-8, wobei die Loop-mediated Isothermal Amplification (LAMP) eine BSM-Polymerase umfasst.

10. Point-of-Care-Verfahren zum Nachweis von Chlamydia trachomatis und/oder Neisseria gonorrhoeae bei einem menschlichen Subjekt ohne Probenreinigung, wobei das Verfahren die folgenden Schritte umfasst:

    a) Bereitstellen einer mehr als 50 % verdünnten Urinprobe von dem menschlichen Subjekt,
    b) Zusetzen eines Cecropin-Peptids zu der Urinprobe,
    c) Amplifizieren der freigesetzten Nukleinsäuren durch Loop-mediated Isothermal Amplification (LAMP) unter Verwendung von Primern, die auf Nukleinsäuren von Chlamydia trachomatis und/oder Neisseria gonorrhoeae abzielen, unter Verwendung einer BSM-Polymerase,
    d) Nachweisen eines Signals von der Nukleinsäure, die von Chlamydia trachomatis und/oder Neisseria gonorrhoeae stammt, durch einen Lateral-Flow-Strip und
    e) Anzeigen, dass das menschliche Subjekt mit Chlamydia trachomatis und/oder Neisseria gonorrhoeae infiziert ist, wenn das Signal über einem vorbestimmten Wert liegt.

    wobei das Point-of-Care-Verfahren eine Nachweisempfindlichkeit von mehr als 60 % im Vergleich zum Cobas®4800 CT/NG Test (Roche) bei Standardeinstellungen aufweist.

11. Point-of-Care-Verfahren zum Nachweis von Chlamydia trachomatis und/oder Neisseria gonorrhoeae bei einem menschlichen Subjekt ohne Probenreinigung, wobei das Verfahren die folgenden Schritte umfasst:

    a) Bereitstellen einer Urinprobe,
    b) Zusetzen eines Cecropin-Peptids zu der Urinprobe und Zulassen, dass das Peptid die Zellen für weniger als 15 Minuten lysiert,
    c) Verdünnen der in Schritt b) erhaltenen Urinprobe zu mehr als 50 % c) Amplifizieren der freigesetzten Nukleinsäuren durch Loop-mediated Isothermal Amplification (LAMP) unter Verwendung von Primern, die auf Nukleinsäuren von Chlamydia trachomatis und/oder Neisseria gonorrhoeae abzielen, unter Verwendung einer BSM-Polymerase,

d) Nachweisen eines Signals von der Nukleinsäure, die von Chlamydia trachomatis und/oder Neisseria gonorrhoeae stammt, durch einen Lateral-Flow-Strip und

e) Anzeigen, dass das menschliche Subjekt mit Chlamydia trachomatis und/oder Neisseria gonorrhoeae infiziert ist, wenn das Signal über einem vorbestimmten Wert liegt,

wobei das Point-of-Care-Verfahren eine Nachweisempfindlichkeit von mehr als 60 % im Vergleich zum Cobas®4800 CT/NG Test (Roche) bei Standardeinstellungen aufweist.

**12.** Verfahren nach einem der Ansprüche 1-11, wobei die auf antimikrobiellem Peptid basierte Freisetzung von Nukleinsäuren mit Tensiden kombiniert wird.

**13.** Verfahren nach einem der Ansprüche 1-12, wobei die Primer aus der Gruppe bestehend aus SEQ ID NO 5-28 ausgewählt sind.

**14.** Teilesatz zum gleichzeitigen Nachweis und zur Quantifizierung von sexuell übertragbaren Krankheiten, wie Chlamydia trachomatis und/oder Neisseria gonorrhoeae, ohne darauf beschränkt zu sein, wobei der Satz umfasst:

a) ein Lysemischung in den folgenden Bereichen: 0,5 - 5 μM AMP (Cecropin P1)

5 - 50 mM EDTA
0,1 - 10 % nichtionisches Tensid

b) eine Mischung, umfassend Nukleotidsequenzen, die für eine Nukleotidsequenz codieren, die aus der Gruppe umfassend beliebige Kombinationen von SEQ ID NO 5-28 ausgewählt ist, oder ein Nukleotid mit 90 % Sequenzidentität mit einer beliebigen von SEQ ID NO: 5-28, und optional eine Polymerase, die in der Lage ist, eine Loop-mediated Isothermal Amplification (LAMP) zu vermitteln, und

c) eine Vorrichtung, die einen Lateral-Flow-Strip umfasst.

## Revendications

**1.** Procédé de détection d'un agent pathogène infectieux sexuellement transmissible chez un sujet humain sans purification d'échantillon, le procédé comprenant les étapes de :

a) fourniture d'un échantillon d'urine provenant du sujet humain,

b) ajout d'un peptide cécropine dans l'échantillon d'urine,

c) amplification des acides nucléiques libérés par amplification isotherme à médiation par boucle (LAMP) à l'aide d'amorces ciblant les acides nucléiques d'agent pathogène infectieux sexuellement transmissible,

d) détection d'un signal de l'acide nucléique provenant de l'organisme d'agent pathogène infectieux sexuellement transmissible, et

e) indication au sujet humain qu'il est infecté par un agent pathogène infectieux sexuellement transmissible, si le signal est supérieur à une valeur prédéterminée.

**2.** Procédé selon la revendication 1, dans lequel l'échantillon d'urine est dilué à plus de 50 % avec un tampon.

**3.** Procédé selon l'une quelconque des revendications 1 et 2, dans lequel le temps de dosage total est inférieur à 60 minutes.

**4.** Procédé selon l'une quelconque des revendications 1 à 3, dans lequel la libération d'acides nucléiques à base de peptides antimicrobiens est combinée à un traitement thermique.

**5.** Procédé selon l'une quelconque des revendications 1 à 4, dans lequel la libération d'acides nucléiques à base de peptides antimicrobiens est combinée à un ou plusieurs tensioactifs.

**6.** Procédé selon l'une quelconque des revendications 1 à 5, dans lequel les réactions LAMP sont analysées dans une bande à écoulement latéral.

7. Procédé selon l'une quelconque des revendications 1 à 6, dans lequel les amorces d'agent pathogène infectieux sexuellement transmissible sont destinées à détecter des acides nucléiques spécifiques de Chlamydia trachomatis.

8. Procédé selon l'une quelconque des revendications 1 à 7, dans lequel la sensibilité est supérieure à 60 %.

9. Procédé selon l'une quelconque des revendications 1 à 8, dans lequel l'amplification isotherme à médiation par boucle (LAMP) comprend une polymérase Bsm.

10. Procédé d'examen de biologie médicale délocalisée pour la détection de Chlamydia trachomatis et/ou de Neisseria gonorrhoeae chez un sujet humain, sans purification d'échantillon, le procédé comprenant les étapes de :

a) fourniture d'un échantillon d'urine dilué à plus de 50 % provenant du sujet humain,
b) ajout d'un peptide cécropine dans l'échantillon d'urine,
c) amplification des acides nucléiques libérés par amplification isotherme à médiation par boucle (LAMP) à l'aide d'amorces ciblant les acides nucléiques de Chlamydia trachomatis et/ou de Neisseria gonorrhoeae à l'aide d'une polymérase Bsm,
d) détection d'un signal de l'acide nucléique provenant de Chlamydia trachomatis et/ou de Neisseria gonorrhoeae par une bande à écoulement latérale, et
e) indication au sujet humain qu'il est infecté par Chlamydia trachomatis et/ou Neisseria gonorrhoeae, si le signal est supérieur à une valeur prédéterminée,

dans lequel le procédé d'examen de biologie médicale délocalisée présente une sensibilité de détection supérieure à 60 % par rapport au test Cobas®4800 CT/NG (Roche) dans des réglages standards.

11. Procédé d'examen de biologie médicale délocalisée pour la détection de Chlamydia trachomatis et/ou de Neisseria gonorrhoeae chez un sujet humain sans purification d'échantillon, le procédé comprenant les étapes de :

a) fourniture d'un échantillon d'urine,
b) ajout d'un peptide cécropine dans l'échantillon d'urine, et le fait de laisser le peptide lyser les cellules pendant moins de 15 minutes,
c) dilution de l'échantillon d'urine obtenu à l'étape b) à plus de 50 %, c) amplification des acides nucléiques libérés par amplification isotherme à médiation par boucle (LAMP) à l'aide d'amorces ciblant les acides nucléiques de Chlamydia trachomatis et/ou de Neisseria gonorrhoeae à l'aide d'une polymérase Bsm,
d) détection d'un signal de l'acide nucléique provenant de Chlamydia trachomatis et/ou de Neisseria gonorrhoeae par une bande à écoulement latérale, et
e) indication au sujet humain qu'il est infecté par Chlamydia trachomatis et/ou Neisseria gonorrhoeae, si le signal est supérieur à une valeur prédéterminée,

dans lequel ledit procédé d'examen de biologie médicale délocalisée présente une sensibilité de détection supérieure à 60 % par rapport au test Cobas®4800 CT/NG (Roche) dans des réglages standards.

12. Procédé selon l'une quelconque des revendications 1 à 11, dans lequel la libération d'acides nucléiques à base de peptides antimicrobiens est combinée à des tensioactifs.

13. Procédé selon l'une quelconque des revendications 1 à 12, dans lequel les amorces sont choisies dans le groupe constitué par SEQ ID NO 5 à 28.

14. Kit de pièces pour la détection et la quantification simultanées de maladies sexuellement transmissibles telles que, mais sans s'y limiter, Chlamydia trachomatis et/ou Neisseria gonorrhoeae, ledit kit comprenant

a) un mélange de lyse dans les plages suivantes :

0,5 - 5 μM d'AMP (Cécropine P1)
5 - 50 mM d'EDTA
0,1 à 10 % de tensioactif non ionique

b) un mélange comprenant des séquences nucléotidiques qui codent pour une séquence nucléotidique choisie dans le groupe comprenant toute combinaison de SEQ ID NO 5 à 28, ou un nucléotide ayant 90 % d'identité de séquence avec l'une quelconque des SEQ ID NO : 5 à 28, et éventuellement une polymérase capable de médier une amplification isotherme à médiation par boucle (LAMP), et

c) un dispositif comprenant une bande à écoulement latéral.

Figure 1

Figure 2A

| percentage of urine | |
|---|---|
| 0% | |
| 5% | |
| 10% | |
| 15% | |
| 20% | |

Figure 2B

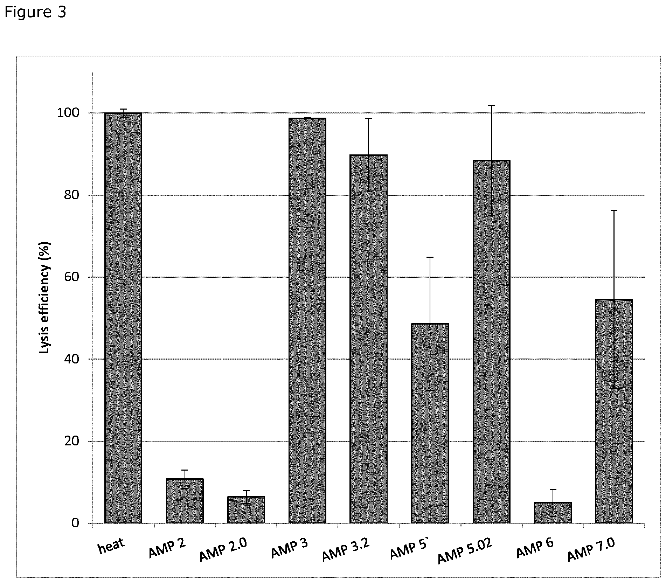

Figure 3

Figure 4

Figure 5A

Figure 5B

| | 0% | 5% | 10% | 15% | 20% |
|---|---|---|---|---|---|
| ▦ Series1 | 21,00 | 19,13 | 18,97 | 22,09 | 28,40 |

Figure 6

| | Untreated | 95C 5 min | Alkaline lysis | lysozyme + detergent | detergent | Cecropin P1 | SB-37 | Melittin | Bombolitin III | MSI-78 | MSI-594 |
|---|---|---|---|---|---|---|---|---|---|---|---|
| Series1 | 100% | 293% | 189% | 86% | 111% | 584% | 167% | 31% | 32% | 16% | 22% |

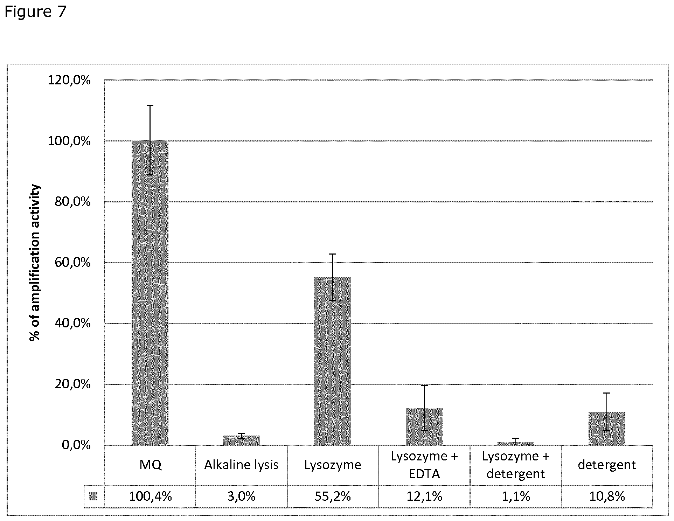

Figure 7

Figure 8

| | MQ | Melittin | Cecropin P1 | SB-37 | Bombolitin III | MSI-78 | MSI-594 |
|---|---|---|---|---|---|---|---|
| | 100,5% | 1,9% | 34,9% | 10,6% | 13,2% | 0% | 0% |

Figure 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9811259 A
- WO 2011095888 A
- WO 2014060604 A
- WO 2011038197 A
- EP 2013071906 W

**Non-patent literature cited in the description**

- **ZWEIG, M. H. ; CAMPBELL, G.** *Clin. Chem.,* 1993, vol. 39, 561-577